# EUROPÄISCHE PATENTSCHRIFT

(11) **EP 3 593 657 B1**
(45) Veröffentlichungstag und Bekanntmachung des Hinweises auf die Patenterteilung: **23.11.2022**
(21) Anmeldenummer: 19181228.8
(22) Anmeldetag: 19.06.2019
(51) Int. Cl.: A24F 47/00, A61M 11/04, A61M 15/02, A61M 15/06

(54) **VERDAMPFEREINHEIT FÜR EINEN INHALATOR, INSBESONDERE FÜR EIN ELEKTRONISCHES ZIGARETTENPRODUKT**
EVAPORATOR UNIT FOR AN INHALER, IN PARTICULAR FOR AN ELECTRONIC CIGARETTE PRODUCT
UNITÉ D'ÉVAPORATEUR POUR UN INHALATEUR, EN PARTICULIER POUR UN PRODUIT DE CIGARETTE ÉLECTRONIQUE

(30) Priorität: 09.07.2018 DE 102018116549; 08.11.2018 DE 102018127926
(43) Veröffentlichungstag der Anmeldung: 15.01.2020
(73) Patentinhaber: Hauni Maschinenbau GmbH, 21033 Hamburg (DE)
(72) Erfinder: Schmidt, Rene, 21244 Buchholz i.d.N. (DE); Niebuhr, Gunnar, 22605 Hamburg (DE); Cornils, Lasse, 22111 Hamburg (DE)
(74) Vertreter: Müller Verweyen

(56) Entgegenhaltungen:
- WO-A1-2019/072915
- WO-A1-2019/072969
- WO-A1-2019/072971
- DE-A1-102014 114 133

## Beschreibung

Die vorliegende Erfindung betrifft einen Verdampferkopf für einen Inhalator, insbesondere für ein elektronisches Zigarettenprodukt, umfassend einen Heizkörper-Dochtstruktur-Aufbau aufweisend einen elektrisch betreibbaren Heizkörper mit mindestens einem flüssigkeitsleitenden Durchgangskanal und einer an einer Einlassseite des Heizkörpers angeordneten porösen und/oder kapillaren Dochtstruktur, und eine Trägerplatte zum Halten des Heizkörper-Dochtstruktur-Aufbaus. Die Erfindung betrifft auch eine Verbrauchseinheit, einen Inhalator sowie ein Verfahren zur Herstellung einer gebrauchsfertigen Verbrauchseinheit.

Im Stand der Technik sind Inhalatoren und insbesondere elektronische Zigarettenprodukte basierend auf dem Docht-Wendel-Prinzip bekannt. Einer Verdampfereinheit beziehungsweise einem Verdampferkopf wird Flüssigkeit aus einem Flüssigkeitsspeicher zugegeben, welches von dem Heizkörper verdampft wird und einem Luftstrom als Dampf und/oder Aerosol zugegeben wird. Verdampfereinheiten basierend auf dem Docht-Wendel-Prinzip führen oft beispielsweise zu ungleichmäßiger Verdampfung, Blasenbildung, lokaler Überhitzung, Trockenlaufen und Verschmutzung.

Im Stand der Technik umfassen Inhalatoren entweder einen offenen oder einen geschlossenen Flüssigkeitsspeicher.

Bei den Offen-Tank-Systemen kann ein Nutzer Liquid beziehungsweise Flüssigkeit nachfüllen. Die Kontrolle über die Art des Liquids und die Nutzung obliegt in diesem Fall dem Nutzer. Der Verdampferkopf muss bei regelmäßigem Gebrauch ausgetauscht werden, da vor allem die Dochtstruktur durch Rückstände aufgrund von lokaler Überhitzung in direkter Nähe zum Heizkörper verdreckt und den Geschmack des Aerosols deutlich verändert. Ferner können Inhalatoren mit Offen-Tank-Systemen durch die Verwendung beliebiger Flüssigkeiten unsachgemäß verwendet werden, was zu Gesundheitsrisiken der Konsumenten führen kann.

Diese Nachteile werden von einem Geschlossen-Tank-System beseitigt. Bei einem Geschlossen-Tank-System umfasst eine Kartusche einen mit Liquid beziehungsweise Flüssigkeit befüllten Flüssigkeitsspeicher und einen mit dem Flüssigkeitsspeicher unlösbar verbundenen Verdampferkopf. Die Kartusche wird vom Nutzer aufgebraucht beziehungsweise leergedampft und anschließend entsorgt, auch wenn der Verdampferkopf noch in einem ohne Rückstände behafteten Zustand und/oder funktionstüchtig ist. Die unlösbare Anbindung des Verdampferkopfes an den Flüssigkeitsspeicher erlaubt nur die Entsorgung der gesamten Kartusche.

Die DE 10 2014 114 133 A1 offenbart eine elektrische Zigarette mit einem Verdampfer und einer Hülle, wobei ein Kapillarspeicher vorgesehen ist, der die gesamte Hülle ausfüllt und sich bis zu einer Brennkammer erstreckt. Wo2019/072969 beschreibt ein Verdampfereinheit für einen Inhalator.

Der Erfindung liegt die Aufgabe zugrunde, einen Verdampferkopf mit einer verbesserten Anbindungsmöglichkeit bereitzustellen.

Die Erfindung schlägt zur Lösung der Aufgabe einen Verdampferkopf gemäß dem unabhängigen Anspruch 1 vor.

Erfindungsgemäß weist der Verdampferkopf eine flüssigkeitsleitende Kanüle auf, die einen vorzugsweise vorragenden Einstechbereich aufweist. Die flüssigkeitsleitende Kanüle ist eine einfach handhabbare Möglichkeit, eine hydraulische Anbindung zwischen Verdampferkopf und einem externen Teil, insbesondere einem Flüssigkeitstank beziehungsweise einem Flüssigkeitsspeicher, herzustellen. Mit der flüssigkeitsleitenden Kanüle kann der Flüssigkeitsspeicher angezapft und Flüssigkeit dem Verdampferkopf zugeführt werden. Durch die Kanüle ist Flüssigkeit aus dem Flüssigkeitsspeicher von dem Einstechbereich zu dem Heizkörper-Dochtstruktur-Aufbau förderbar, um die Flüssigkeit aus dem Flüssigkeitsspeicher zur Dochtstruktur und/oder zum Heizkörper fördern zu können. Der Einstechbereich ragt vorteilhaft über den Verdampferkopf, um die Kanüle in ein externes Teil, beispielsweise in einen Flüssigkeitstank, einstechen zu können.

Aufgrund der Erfindung kann der Konsument auf einfache Weise eine gebrauchsfertige Einheit mit Verdampferkopf und Flüssigkeitsspeicher herstellen und den Verdampferkopf auf einfache Weise tauschen. Der Verdampferkopf kann daher auch als Wechselverdampfer bezeichnet werden.

Die erfindungsgemäße Kanüle ist insbesondere rohrförmig, jedoch nicht auf eine spezielle, beispielsweise runde Querschnittsform beschränkt, sondern umfasst alle denkbaren und geeigneten Querschnittsformen.

Die Kanüle umfasst vorteilhaft eine beidseitig offene Röhre, durch die Flüssigkeit transportierbar ist. An der einen offenen Seite beziehungsweise Stichseite weist die Kanüle den Einstechbereich auf und an der anderen, dem Einstechbereich gegenüberliegenden, offenen Seite beziehungsweise Befestigungsseite ist die Kanüle an dem der Trägerplatte und/oder dem Heizkörper-Dochtstruktur-Aufbau befestigt. Zwischen den beiden Seiten, d.h. der Stichseite und der Befestigungsseite erstreckt sich vorteilhaft ein flüssigkeitsleitender Kanal innerhalb der Kanüle, in dem Flüssigkeit vom Einstechbereich beziehungsweise der Stichseite zur Dochtstruktur, zum Heizkörper und/oder zur Befestigungsseite förderbar ist.

Die Kanüle ist an der Befestigungsseite an der Trägerplatte und/oder dem Heizkörper-Dochtstruktur-Aufbau befestigt. Die Befestigung zwischen der Kanüle und der Trägerplatte bzw. dem Heizkörper-Dochtstruktur-Aufbau kann beispielsweise eine Klemmung der Kanüle in einer Durchgangsöffnung der Trägerplatte, Klemmung der Kanüle mit an der Trägerplatte oder dem Heizkörper-Dochtstruktur-Aufbau angeordneten Klemmelementen, Klebung und/oder Verschweißung sein. In einer Ausführungsform sind die Trägerplatte und die Kanüle einteilig ausgebildet, d.h. sie bestehen aus demselben Material und bilden gemeinsam ein einziges Teil aus. Die Kanüle ist vorzugsweise durch den Nutzer unlösbar an der Trägerplatte und/oder dem Heizkörper-Dochtstruktur-Aufbau befestigt.

Die Kanüle schließt mit der vorzugsweise wenigstens lokal planaren Trägerplatte vorteilhaft einen Winkel von beispielsweise 30° bis 90°, vorzugsweise 45° bis 90° und weiter vorzugsweise 90° ein, um eine vorteilhafte Ausrichtung der Kanüle zu gewährleisten.

In einer bevorzugten Ausführungsform ist die Dochtstruktur und/oder der Heizkörper an einer Befestigungsseite der Kanüle angeordnet, um eine zuverlässige Förderung von Flüssigkeit zu erreichen, die Dochtstruktur und/oder den Heizkörper von der Trägerplatte haltbar anordnen zu können und/oder die Zugabe von verdampfter Flüssigkeit als Aerosol beziehungsweise Dampf an der Auslassseite vorteilhaft zu ermöglichen. Die Dochtstruktur kann sich vorteilhaft in die Kanüle hinein erstrecken, beispielsweise mindestens 25%, vorzugsweise mindestens 50%, noch weiter vorzugsweise mindestens 75% bezogen auf die Länge der Kanüle. Die Dochtstruktur kann sich in anderen Ausführungsformen jedoch auch durch die ganze Kanüle erstrecken. In dieser Ausführungsform ist der Heizkörper-Dochtstruktur-Aufbau an einem Ende der Kanüle bzw. Röhre angeordnet, während das andere freie Ende offen bleibt, um Flüssigkeit aus dem Flüssigkeitsspeicher aufnehmen zu können.

Vorteilhaft ist die Dochtstruktur wenigstens teilweise innerhalb der Kanüle angeordnet, um einen effektiven Aufbau des Verdampferkopfes und eine zuverlässige Flüssigkeitsförderung zu ermöglichen. Die Anordnung der Dochtstruktur innerhalb der Kanüle kann auch der vorteilhaften Halterung des Heizkörper-Dochtstruktur-Aufbaus dienen.

Vorzugsweise ist der Heizkörper-Dochtstruktur-Aufbau als ein Schichtaufbau ausgebildet, um eine kompakte Bauweise und eine fehlerfreie Funktion zu fördern. Der Schichtaufbau umfasst den Heizkörper und die Dochtstruktur, wobei die Dochtstruktur an der Einlassseite des Heizkörpers angeordnet ist. Der Heizkörper und die Dochtstruktur bilden jeweils mindestens eine Schicht des Schichtaufbaus. Vorteilhaft kontaktieren der Heizkörper und die Dochtstruktur einander flächig, um den Schichtaufbau insbesondere kompakt und stabil auszubilden und/oder eine kapillare Förderung von Flüssigkeit zu ermöglichen. Flüssigkeit ist vorteilhaft durch die Dochtstruktur über die Einlassseite des Heizkörpers in den Schichtaufbau förderbar und vom Heizkörper verdampfbar. Der Schichtaufbau kann eine Mehrzahl von Schichten umfassen, welche trennbar oder untrennbar zusammengehalten sind. Der Heizkörper und/oder die Dochtstruktur kann wiederum eine Mehrzahl von Schichten umfassen. Der Schichtaufbau kann weitere Schichten, beispielsweise zur Verbindung von Dochtstruktur und Heizkörper, zur Beeinflussung des Flüssigkeitstransports und/oder zur mechanischen Stabilisierung, umfassen.

Es ist von Vorteil, dass die Kanüle einen sich zwischen einer Befestigungsseite und einer Stichseite erstreckenden Mantel aufweist, wobei der Mantel wenigstens teilweise den Heizkörper-Dochtstruktur-Aufbau umfänglich, insbesondere abschließend, ummantelt. Der Mantel weist eine Innenseite auf und begrenzt den flüssigkeitsleitenden Kanal der Kanüle.

Der Heizkörper-Dochtstruktur-Aufbau kontaktiert vorteilhaft den Mantel, wobei der Heizkörper-Dochtstruktur-Aufbau mindestens teilweise an der Innenseite des Mantels anliegen kann. Der Heizkörper-Dochtstruktur-Aufbau kontaktiert den Mantel vorteilhaft umfänglich, um einen kontrollierten Flüssigkeitstransport zu erlauben. Die Kanüle weist eine sich zwischen der Befestigungsseite und der Stichseite erstreckende Längsachse auf. Der Mantel erstreckt sich röhrenförmig oder hohlzylinderförmig parallel zur Längsachse.

Erfindungsgemäß ist der Einstechbereich zum Einstechen in ein externes Teil, insbesondere in einen Flüssigkeitsspeicher ausgebildet, um eine einfache hydraulische beziehungsweise flüssigkeitsleitende Anbindung an ein externes Teil, insbesondere einen Flüssigkeitsspeicher beziehungsweise einen Flüssigkeitstank bereitstellen zu können.

Vorteilhaft weist der Einstechbereich eine Schneide auf, um ein sicheres, zuverlässiges und sauberes Eindringen, Einstechen beziehungsweise Einschneiden der Kanüle in den Flüssigkeitsspeicher zu ermöglichen. In einer Ausführungsform weist die Kanüle im Einstechbereich eine Spitze auf. Die Kanüle kann auch stumpf ausgebildet sein, um eine Verletzungsgefahr für den Verbraucher zu reduzieren.

Vorzugsweise weist die Trägerplatte eine Durchgangsöffnung auf, in der der Heizkörper-Dochtstruktur-Aufbau und/oder die Kanüle gehalten sein können, wobei die Flüssigkeit von der Kanüle zu dem Heizkörper durch die Durchgangsöffnung förderbar ist. Beispielsweise ist der Heizkörper mindestens teilweise innerhalb der Durchgangsöffnung angeordnet. In diesem Fall kann die Auslassseite des Heizkörpers mit der Trägerplatte fluchten. In einem anderen Beispiel ragt die Auslassseite des Heizkörpers aus der Trägerplatte heraus.

Bevorzugt erstreckt sich der Heizkörper-Dochtstruktur-Aufbau mindestens teilweise durch die Durchgangsöffnung der Trägerplatte, um den Heizkörper-Dochtstruktur-Aufbau in der Trägerplatte halten zu können und eine vorteilhafte Ausrichtung und/oder Anordnung der Dochtstruktur und/oder des Heizkörpers, insbesondere der Auslassseite, zu erlauben.

Es ist von Vorteil, dass der Verdampferkopf ein Arretierungselement zur Arretierung in einem externen Teil, insbesondere in einem Flüssigkeitsspeicher umfasst, um den Verdampferkopf mit dem Flüssigkeitsspeicher zu einer gebrauchsfertigen Einheit verbinden zu können. Das Arretierungselement kann beispielsweise magnetisch sein und Magnete umfassen. Das Arretierungselement kann mechanisch sein und beispielsweise eine(n) Clip, Rastelement, Klemme, Klammer, Feder, Gewinde und/oder ein anderes mechanisches Arretierungselement umfassen.

Das Arretierungselement kann durch den Konsumenten zerstörungsfrei lösbar sein. In dieser Ausführungsform kann der Konsument den Verdampferkopf von dem Flüssigkeitsspeicher ohne Beschädigung trennen, um beispielsweise den Verdampferkopf wechseln und/oder reinigen zu können. Der Verdampferkopf beziehungsweise Wechselverdampfer kann beispielsweise mittels einer insbesondere alkoholbasierten Lösung oder durch Pyrolyse gereinigt werden, abhängig davon, wie oft der Verdampferkopf verwendet wurde und ob ein Aroma- und/oder Wirkstoffwechsel stattfindet. Nach der Reinigung kann der Verdampferkopf an einen neuen beziehungsweise anderen Flüssigkeitsspeicher angeschlossen werden.

Das Arretierungselement kann durch den Konsumenten unlösbar sein, um ein einmaliges Montieren einer gebrauchsfertigen Einheit für den Konsumenten, beispielsweise durch den Hersteller, zu ermöglichen.

Vorzugsweise besteht die Kanüle aus Metall, um eine effektive, geschmacksneutrale und standardisierte Ausführungsform des Verdampferkopfes bereitstellen zu können.

Bevorzugt ist ein Mundstück an dem Verdampferkopf, insbesondere an der Trägerplatte, befestigbar oder befestigt, um den Nutzer die Handhabung und/oder Reinigung des Verdampferkopfes und/oder des Mundstücks zu erleichtern. Der Verdampferkopf kann ein Arretierungselement zur Arretierung des Mundstücks aufweisen. Die Kanüle und/oder der Heizkörper-Dochtstruktur-Aufbau kann vorteilhaft so angeordnet sein, dass die Kanüle und/oder der Heizkörper-Dochtstruktur-Aufbau im gebrauchsfertigen Zustand in dem Mundstück (insbesondere vollständig) versenkt sind, um beispielsweise eine Verletzung durch die Kanüle und/oder eine Verschmutzung des Heizkörper-Dochtstruktur-Aufbaus zu vermeiden.

Vorteilhaft ist der Heizkörper ein Mikromechanisches System (MEMS) und/oder siliziumbasiert, um eine standardisierte und effektive Herstellung zu erlauben und eine hohe Dampf- beziehungsweise Aerosolqualität bereitstellen zu können.

Vorteilhaft ist ein Tankgehäuse des Flüssigkeitsspeichers von einer Kanüle durchstechbar ausgebildet, um einen vor dem Durchstechen mit der Kanüle geschlossenen, flüssigkeitsdichten Flüssigkeitsspeicher an den Verdampferkopf flüssigkeitsleitend und vorteilhaft flüssigkeitsdicht anbinden zu können. Vorteilhaft enthält der Flüssigkeitsspeicher vor der Verbindung mit dem Verdampferkopf eine herstellerseitig vorgegebene Flüssigkeit, um einen Missbrauch des Inhalators vorzubeugen. Der Flüssigkeitsspeicher ist vorteilhaft in einem bestimmbaren, der Kanüle zuordenbaren Einstechabschnitt dazu eingerichtet, von der Kanüle durchstochen zu werden.

Vorteilhaft kann der Flüssigkeitsspeicher eine Dichtung, insbesondere im Bereich des Einstechabschnitts, aufweisen, die das Entfernen der Kanüle nach dem Einstechen erlauben, wobei der Flüssigkeitsspeicher nach dem Entfernen flüssigkeitsdicht ist. In dieser Ausführungsform kann der Konsument beispielsweise zur späteren Verwendung der Flüssigkeit in dem Flüssigkeitsspeicher und/oder zur Reinigung des Verdampferkopfes den Flüssigkeitsspeicher vom Verdampferkopf trennen.

Vorzugsweise umfasst das Tankgehäuse des Flüssigkeitsbehälters ein Einstechsiegel, um die Unversehrtheit des Flüssigkeitsspeichers beispielsweise vor einem Einstich der Kanüle feststellen zu können. Das Einstechsiegel ist vorteilhaft dazu eingerichtet, beim Einstechen der Kanüle in den Flüssigkeitsbehälter zerstört zu werden. Vorteilhaft indiziert das Einstechsiegel die Authentizität des Flüssigkeitsspeichers und insbesondere der darin enthaltenen Flüssigkeit mit den Aroma- und/oder Wirkstoffen. In einer Ausführungsform kann das Einstechsiegel nach dem Einstich der Kanüle lesbar bleiben. Das Einstichsiegel kann beispielsweise ein Logo, eine Nummer, eine Prüfziffer, einen scanbaren Code, beispielsweise einen Strich- oder zweidimensionalen Code und/oder andere durch Menschen und/oder Maschinen lesbare Elemente umfassen.

Bevorzugt ist der Flüssigkeitsspeicher wenigstens teilweise aus von einer Kanüle durchstechbarem Kunststoff und/oder Metall, insbesondere Aluminium, gebildet, um eine einfache Konstruktion und Herstellung des Flüssigkeitsspeichers zu erlauben. Der Flüssigkeitsspeicher kann beispielsweise ein Beutel aus Kunststoff sein oder einen solchen umfassen. Das Einstechsiegel besteht vorteilhaft aus einem von einer Kanüle durchstechbaren Material, das gleich oder verschieden von dem Material des restlichen Tankgehäuses sein kann. Beispielsweise kann das Tankgehäuse aus Kunststoff sein und das Einstechsiegel umfasst eine Aluminium-Kappe, Aluminium-Folie oder eine Kunststofffolie, in die die am Verdampferkopf befestigte Kanüle eingestochen werden kann. In einem Ausführungsbeispiel ist der Flüssigkeitsspeicher als Hohlzylinder, insbesondere aus einem Kunststoff, ausgeführt.

Es ist von Vorteil, dass der Flüssigkeitsspeicher eine Arretierungseinrichtung zur Arretierung, insbesondere durch eine Drehbewegung, eines externen Teils, insbesondere eines Verdampferkopfes, umfasst. Die Arretierungseinrichtung des Flüssigkeitsspeichers ist vorteilhaft dazu eingerichtet, mit dem Arretierungselement des Verdampferkopfes zusammenzuwirken, damit der Flüssigkeitsspeicher und der Verdampferkopf eine gebrauchsfertige Einheit bilden können. Vorzugsweise wird mit der Arretierung eine flüssigkeitsdichte Verbindung zwischen dem Flüssigkeitsspeicher und dem externen Teil hergestellt. In einer Ausführungsform ist der Arretierungseinrichtung wenigstens eine Dichtung beziehungsweise ein Dichtelement zur flüssigkeitsdichten Abdichtung zugeordnet, um eine flüssigkeitsdichte Arretierung des externen Teils zu erreichen.

Die Arretierungseinrichtung kann beispielsweise magnetisch sein und/oder Magnete umfassen. Die Arretierungseinrichtung kann mechanisch sein und beispielsweise eine(n) Clip, Rastelement, Klemme, Klammer, Feder, Gewinde und/oder eine andere mechanische Arretierungseinrichtung umfassen.

Die Arretierungseinrichtung kann durch den Konsumenten zerstörungsfrei lösbar sein. In dieser Ausführungsform kann der Konsument den Verdampferkopf von dem Flüssigkeitsspeicher ohne Beschädigung trennen, um den Verdampferkopf wechseln und/oder reinigen zu können.

Die Arretierungseinrichtung kann durch den Konsumenten unlösbar sein, um ein einmaliges Montieren einer gebrauchsfertigen Einheit für den Konsumenten, beispielsweise durch den Hersteller, zu ermöglichen.

Vorteilhaft ist der Verdampferkopf mit der Kanüle in den Flüssigkeitsspeicher zur Herstellung einer gebrauchsfertigen Einheit einstechbar, um auf eine einfache und sichere Art die gebrauchsfertige Verbrauchseinheit umfassend mindestens den Verdampferkopf und den Flüssigkeitsspeicher herstellen zu können. In einer Ausführungsform kann die Verbrauchseinheit als Kartusche in den Inhalator einsetzbar sein, beispielsweise in eine zur Aufnahme der Verbrauchseinheit vorgesehene Öffnung des Inhalators, beispielsweise an der Seite des Inhalatorgehäuses.

Vorteilhaft weist die Verbrauchseinheit ein Mundstück auf, damit die Verbrauchseinheit für den Nutzer vorteilhaft handhabbar ist.

Vorteilhaft ist die Trägerplatte des Verdampferkopfes an dem Mundstück befestigt. Die Befestigung des Verdampferkopfes an dem Mundstück kann von dem Konsumenten zerstörungsfrei lösbar, oder alternativ unlösbar sein. Das Mundstück kann in einer Ausführungsform einen Teil des Inhalatorgehäuses des betriebsfertigen Inhalators ausbilden.

Vorteilhaft ist die Kanüle im gebrauchsfertigen Zustand in dem Mundstück, insbesondere vollständig, versenkt, um eine Verletzungsgefahr des Konsumenten durch die Kanüle zu vermeiden. Das Mundstück kann einen hohlzylinderförmigen Abschnitt aufweisen, innerhalb dessen die Kanüle angeordnet ist. Vorzugsweise ist die Kanüle entlang einer Längsachse des hohlzylinderförmigen Abschnitts des Mundstücks angeordnet, wobei sich das Mundstück in dem hohlzylinderförmigen Abschnitt koaxial zu einer Längsachse der Kanüle verläuft. Der Flüssigkeitsspeicher ist dazu eingerichtet beziehungsweise so bemessen, in den hohlzylinderförmigen Abschnitt des Mundstücks eingeführt und dabei von der Kanüle durchstochen zu werden. Der Begriff zylindrisch ist nicht auf einen kreisrunden Querschnitt beschränkt, sondern kann beliebige geeignete Querschnitte umfassen.

Vorzugsweise fluchtet die Trägerplatte im gebrauchsfertigen Zustand mit dem Tankgehäuse des Flüssigkeitsspeichers, um eine gute Einbaubarkeit in ein Inhalatorgehäuse eines Inhalators zu ermöglichen und/oder eine Kartusche ohne den Nutzer störenden Kanten bereitstellen zu können.

Vorteilhaft schließt die Trägerplatte im gebrauchsfertigen Zustand den Flüssigkeitstank flüssigkeitsdicht ab, um den Konsumenten im Gebrauch eine flüssigkeitsdichte Einheit bereitstellen zu können.

Bevorzugt ergänzt die Trägerplatte das Tankgehäuse des Flüssigkeitsspeichers im gebrauchsfertigen Zustand zu einem vollständigen und flüssigkeitsdichten Flüssigkeitstank, um den Flüssigkeitsspeicher nach dem Durchstich mit der Kanüle als flüssigkeitsdichte Einheit bereitstellen zu können.

Ein einfaches und zuverlässiges Verfahren zur Herstellung der gebrauchsfertigen Verbrauchseinheit für den Inhalator umfasst die Bereitstellung eines Verdampferkopfes, die Bereitstellung eines Flüssigkeitsspeichers und das Einstechen der Kanüle des Verdampferkopfes in das Tankgehäuse des Flüssigkeitsspeichers.

Vorzugsweise umfasst das Verfahren die Arretierung des Verdampferkopfes am Flüssigkeitsspeicher, insbesondere durch eine Drehbewegung, beispielsweise in der Art eines Bajonettverschlusses, um das Verfahren durch eine definierte, vom Nutzer wahrnehmbare Arretierung abschließen zu können. Vorteilhaft erfolgt mit der Arretierung das Einstechen der Kanüle des Verdampferkopfes in das Tankgehäuse des Flüssigkeitsspeichers.

Die Erfindung wird im Folgenden anhand bevorzugter Ausführungsformen unter Bezugnahme auf die beigefügten Figuren erläutert. Dabei zeigt
- Fig. 1: eine schematische Querschnittsansicht einer Verbrauchseinheit mit einem Mundstück;
- Fig. 2: eine perspektive Querschnittsansicht einer Verbrauchseinheit;
- Fig. 3: eine schematische Darstellung eines Inhalators;
- Fig. 4: eine schematische Querschnittsansicht eines Schichtaufbaus;
- Fig. 5: eine perspektivische Querschnittsansicht eines Schichtaufbaus;
- Fig. 6: eine schematische Querschnittsansicht eines Schichtaufbaus in einer Ausführungsform der Erfindung; und
- Fign. 7, 8: perspektivische Aufsichten auf eine Trägerplatte von der Seite eines Heizkörpers bzw. von der entgegensetzten Seite einer Flüssigkeitszuführung.

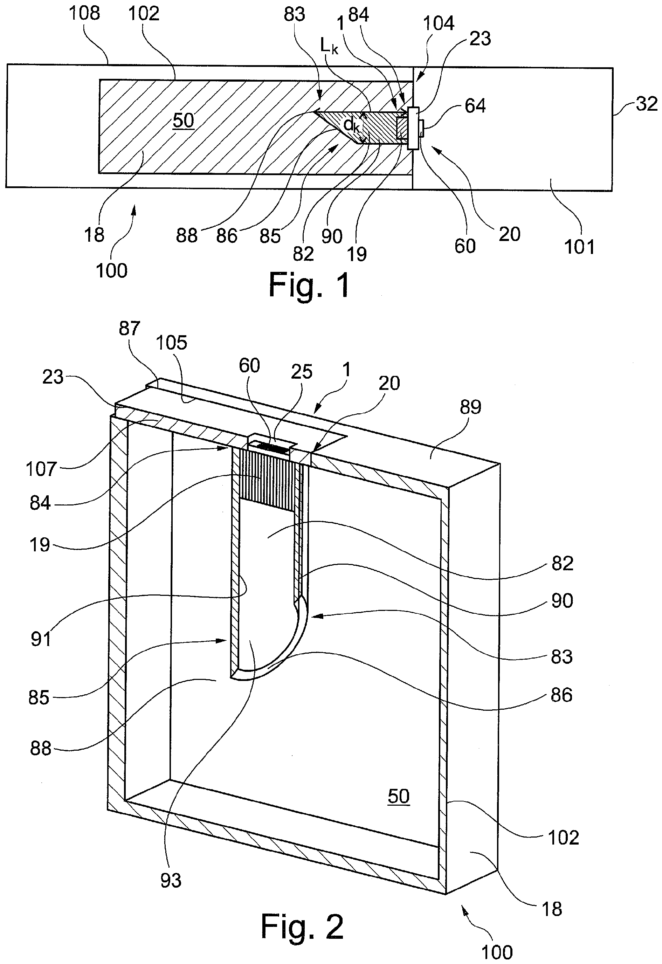

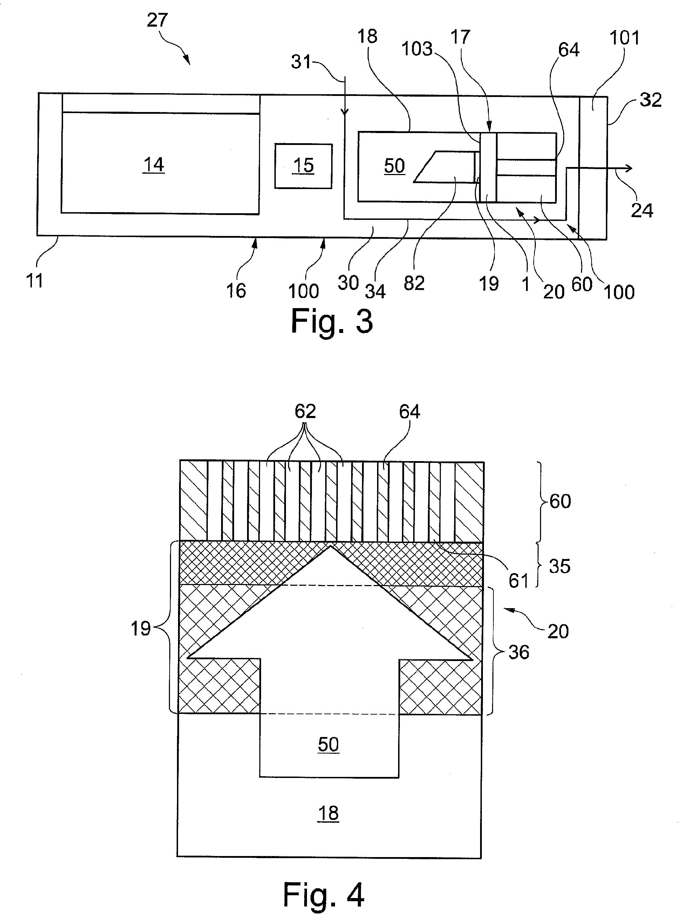

Figur 1 zeigt eine gebrauchsfertige Verbrauchseinheit 100 für einen beispielsweise in Figur 3 gezeigten Inhalator 27. Die in Figur 1 gezeigte Verbrauchseinheit 100 umfasst einen Flüssigkeitsspeicher 18, einen Verdampferkopf 1 und ein Mundstück 101. Die Verbrauchseinheit 100 kann eine Wechselkartusche 17 ausbilden. Der Flüssigkeitsspeicher 18 kann vorteilhaft von dem Nutzer wechselbar sein und/oder der Verdampferkopf 1 und/oder das Mundstück 101 können wiederverwendbar sein.

Der Verdampferkopf 1 umfasst einen als Schichtaufbau ausgebildeten Heizkörper-Dochtstruktur-Aufbau 20 aus einem elektrisch betreibbaren Heizkörper 60 und einer porösen und/oder kapillaren Dochtstruktur 19. Flüssigkeit 50 aus dem Flüssigkeitsspeicher 18 ist dem Heizkörper 60 über die Dochtstruktur 19 zuführbar. Der Heizkörper 60 ist dazu eingerichtet, die ihm zugeführte Flüssigkeit 50 zu verdampfen und einem Luftstrom 34 (Figur 3) als Aerosol beziehungsweise Dampf zuzugeben. Der Verdampferkopf 1 umfasst eine Trägerplatte 23 zum Halten des Schichtaufbaus 20, so dass die Trägerplatte 23 und der Schichtaufbau 20 miteinander verbunden sind. Beispielhafte Ausführungsformen eines Schichtaufbaus 20 sind jeweils den Figuren 4 bis 6 zu entnehmen.

Der Verdampferkopf 1 in Figur 1 weist eine flüssigkeitsleitende Kanüle 82 auf, die an einer Befestigungsseite 84 der Kanüle 82 an der Trägerplatte 23 und/oder dem Heizkörper-Dochtstruktur-Aufbau 20 befestigt ist.

Vorteilhaft ist die Kanüle 82 röhrenförmig. Zwischen einer Stichseite 85 und der Befestigungsseite 84 ist vorteilhaft eine Längsachse definiert. Der Mantel 90 erstreckt sich vorteilhaft parallel und koaxial zur Längsachse über eine Länge Lk. Der Mantel 90 definiert einen flüssigkeitsleitenden Kanal 93 der Kanüle 82 und weist einen Umfang beziehungsweise einen Durchmesser dk oder eine andere typische Ausdehnung senkrecht zur Längsachse, beispielsweise eine Kantenlänge, auf.

Die Kanüle 82 ist vorteilhaft länglich, d.h. der Durchmesser dk ist kleiner als die Länge Lk entlang der Längsachse der Kanüle 82. Der Außendurchmesser dk der Kanüle beträgt vorteilhaft zwischen 0,25 mm und 3,4 mm, vorzugsweise zwischen 0,5 mm und 1,8 mm. Die Länge Lk der Kanüle 82 beträgt vorteilhaft zwischen 1 mm und 120 mm, vorzugsweise zwischen 2 mm und 50 mm, weiter vorzugsweise zwischen 3 mm und 10 mm. Die Kanüle 82 ist vorteilhaft in der Art einer Spritzenkanüle beziehungsweise Injektionskanüle ausgestaltet. In diesem Beispiel ist die Trägerplatte 23 planar und die Kanüle 82, insbesondere deren Längsachse, schließt mit der Oberfläche der Trägerplatte 23 einen rechten Winkel ein.

Die Kanüle 82 umfasst an der Stichseite 85 vorteilhaft einen Einstechbereich 83, der eine Schneide 86 und/oder eine Spitze 88 aufweisen kann. Die Schneide 86 erstreckt sich vorteilhaft von einem der Stichseite 85 zugeordneten Ende eines röhrenförmigen beziehungsweise hohlzylinderförmigen Abschnitts der Kanüle 82 bis zur Spitze 88 der Kanüle 82. Die Spitze 88 ist dazu eingerichtet, in den Flüssigkeitstank 50 einzudringen, einzustechen oder einzuschneiden. Die Schneide 86 schneidet vorteilhaft einen definierten Schnitt in den Flüssigkeitstank 50, wobei die Abmessungen des Schnitts den Abmessungen der Kanüle 82, insbesondere der Dicke der Kanüle 82 entspricht.

Die Dochtstruktur 19 und der Heizkörper 60 sind vorteilhaft an der Befestigungsseite 84 angeordnet. Vorteilhaft umschließt beziehungsweise ummantelt die Kanüle 82 die Dochtstruktur 19, d.h. die Dochtstruktur 19 liegt zumindest teilweise in dem Kanal 93 der Kanüle 82. Der Heizkörper 60 ist vorteilhaft wenigstens teilweise außerhalb der Kanüle 82 angeordnet, so dass eine Auslassseite 64 des Heizkörpers 60 in das in Figur 1 gezeigte Mundstück 101 hineinragt, um dort von dem Luftstrom 34 umströmt werden zu können. Die Dochtstruktur 19 erstreckt sich ausgehend von der Befestigungsseite 84 innerhalb der Kanüle 82 entlang der Längsachse der Kanüle 82 in Richtung der Stichseite 85.

Das Mundstück 101 und der Schichtaufbau 20 sind vorteilhaft so angeordnet, dass der Nutzer bei sachgemäßem Gebrauch nicht in Berührung mit dem Schichtaufbau 20 kommen kann, um den Schichtaufbau 20 vor Verschmutzung und/oder Beschädigung zu schützen.

Die Verbrauchseinheit 100 weist vorteilhaft eine Umhüllung 108 auf. Vorzugsweise umfasst das Mundstück 101 die Umhüllung 108. Die Kanüle 82 ist vorteilhaft im gebrauchsfertigen Zustand vollständig in dem Mundstück 101 beziehungsweise der Umhüllung 108 versenkt. Die Umhüllung 108 ist vorteilhaft so bemessen, dass der Flüssigkeitstank 50 in die Umhüllung 108 einsetzbar, beispielsweise einschiebbar und/oder eindrehbar ist, um von der Kanüle 82 durchstochen zu werden.

Figur 2 zeigt eine Verbrauchseinheit 100, umfassend einen Flüssigkeitsspeicher 18 mit einem Tankgehäuse 102 und einem Verdampferkopf 1. Die Arretierungseinrichtung 105 umfasst in diesem Beispiel eine Aussparung 107 in dem Tankgehäuse 102 beispielsweise an einer Stirnseite 89 des Tankgehäuses 102. Der Flüssigkeitsspeicher 18 kann im Bereich der Aussparung 107 ein Einstechsiegel 103 aufweisen, das zum Durchstechen mit der Kanüle 82 eingerichtet ist. Beispielsweise kann das Tankgehäuse 102 aus einem im Vergleich zum Einstechsiegel 103 festen Kunststoff gebildet sein und das Einstechsiegel 103 kann eine durchstechbare Folie oder Kappe umfassen. Das Tankgehäuse 102 kann in anderen Ausführungsformen auch ein flexibler Beutel sein.

In dem in Figur 2 gezeigten Zustand ist die Kanüle 82 in den Flüssigkeitsspeicher 18 eingestochen. Der Verdampferkopf 1 ist mittels zusammenwirkender Arretierungselemente 87 und/oder Arretierungseinrichtung 105 mit dem Flüssigkeitsspeicher 18 verbunden und/oder arretiert. Ein Arretierungselement 87 ist vorteilhaft an der Trägerplatte 23 angeordnet. Eine mit dem Arretierungselement 87 zusammenwirkende Arretierungseinrichtung 105 ist vorteilhaft an dem Tankgehäuse angeordnet. Vorteilhaft ist die Trägerplatte 23 so ausgebildet, dass die Form der Trägerplatte 23 der Form der Aussparung 107 des Tankgehäuses 102 entspricht. Die Trägerplatte 23 fluchtet im gebrauchsfertigen Zustand mit dem Tankgehäuse 102 bzw. der Stirnseite 89 des Tankgehäuses 102 und schließt die Aussparung 107 flüssigkeitsdicht ab.

Nach dem Einstich der Kanüle 82 in den Flüssigkeitsspeicher 18 und der Arretierung des Verdampferkopfes 1 am Flüssigkeitstank 18 bildet die Trägerplatte 23 und das Tankgehäuse 102 einen flüssigkeitsdichten Flüssigkeitstank 104.

Nach der Entleerung des Flüssigkeitsspeichers 18 beziehungsweise des Flüssigkeitstanks 104 kann dieser mit einem zerstörungsfrei lösbaren Arretierungselement 87 vom Verdampferkopf 1 getrennt werden, wobei der Flüssigkeitsspeicher 18 beziehungsweise Flüssigkeitstank 104 entsorgt und/oder der Verdampferkopf 1 gereinigt und wiederverwendet, insbesondere mehrfach wiederverwendet, werden kann. Der Flüssigkeitstank 18 ist in diesem Beispiel Teil einer Wechselkartusche 17, oder bildet eine Wechselkartusche 17.

Der Flüssigkeitsspeicher 18 beziehungsweise der Flüssigkeitstank 104 fasst eine Flüssigkeit 50, welche Aroma- und/oder Wirkstoffe, insbesondere Nikotin und/oder medizinische Stoffe, enthält. Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml. Vorzugsweise ist der Flüssigkeitsspeicher 18 beziehungsweise der Flüssigkeitstank 104 vorbefüllt, d.h. vom Hersteller des der Flüssigkeitsspeichers 18 beziehungsweise Flüssigkeitstanks 104 befüllt.

Figur 3 zeigt einen Inhalator 27, hier ein elektronisches Zigarettenprodukt, umfassend ein Inhalatorgehäuse 11, in dem ein Luftkanal 30 zwischen mindestens einer Lufteinlassöffnung 31 und einer Luftauslassöffnung 24 an einem Mundende 32 des Inhalators 27 vorgesehen ist. Das Mundende 32 des Inhalators 27 bezeichnet dabei das Ende, an dem der Konsument zwecks Inhalation zieht und dadurch den Inhalators 27 mit einem Unterdruck beaufschlagt und eine Luftströmung 34 in dem Luftkanal 30 erzeugt.

Der Inhalator 27 besteht vorteilhaft aus einem wiederverwendbaren Basisteil 16 und einer Verbrauchseinheit 100. Die Verbrauchseinheit 100 umfasst den Verdampferkopf 1 und den Flüssigkeitsspeicher 18.

In einer Ausführungsform ist die gebrauchsfertige Einheit aus Verdampferkopf 1 und Flüssigkeitsspeicher 18 in Form einer Kartusche in das Mundstück 101 einsetzbar. Die dadurch gebildete Verbrauchseinheit 100 aus Kartusche und Mundstück 101 ist dann mit dem Basisteil 16 verbindbar, um den gebrauchsfertigen Inhalator 27 zu bilden.

In einer anderen Ausführungsform ist das Mundstück 101 für den Konsumenten unlösbar mit dem Verdampferkopf 1 verbunden. In dieser Ausführungsform wird der Flüssigkeitsspeicher 18 in das Mundstück 101 eingesetzt, wodurch die Kanüle 82 in den Flüssigkeitsspeicher 18 eindringt. Die dadurch gebildete Verbrauchseinheit 100 aus Mundstück 101 mit Verdampferkopf 1 und Flüssigkeitsspeicher ist dann wiederum mit dem Basisteil 16 verbindbar, um den gebrauchsfertigen Inhalator 27 zu bilden.

Das Mundstück 101 kann einen Teil des Inhalatorgehäuses 11 des gebrauchsfertigen Inhalators 27 ausbilden beziehungsweise das Inhalatorgehäuse 11 ergänzen.

In einer Ausführungsform lässt sich die Verbrauchseinheit 100 als gebrauchsfertige Kartusche beziehungsweise Wechselkartusche 17 beispielsweise in eine Öffnung des Inhalatorgehäuses 11 beispielsweise an einer Seite des Inhalators 27 in das Inhalatorgehäuse 11 einsetzen und dort fixieren.

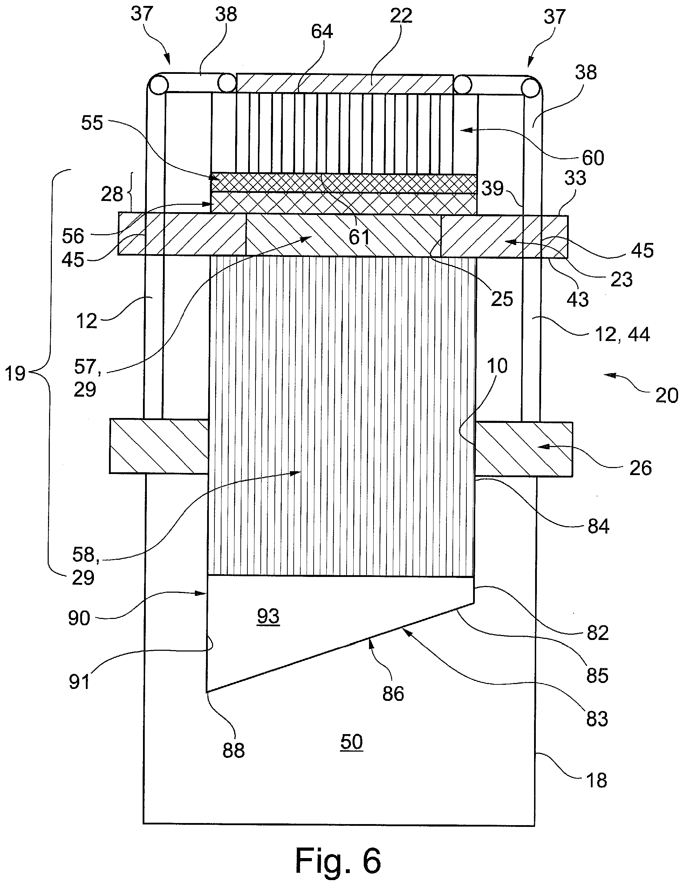

Die durch die Einlassöffnung 31 angesaugte Luft wird in dem Luftkanal 30 zu oder entlang mindestens eines Schichtaufbaus 20 geleitet. Der Schichtaufbau 20 ist mit mindestens einem Flüssigkeitsspeicher 18 verbunden oder verbindbar, in dem mindestens eine Flüssigkeit 50 gespeichert ist. Der Schichtaufbau 20 verdampft Flüssigkeit 50, die ihr aus dem Flüssigkeitsspeicher 18 zugeführt wird, und gibt die verdampfte Flüssigkeit als Aerosol/Dampf 22 (siehe Figur 6) an einer Auslassseite 64 in den Luftstrom 34 zu. Die Luftzuführung am Schichtaufbau 20 beziehungsweise dem Verdampfer vorbei zum Mundstück 101 wird in einer Ausführungsform vorteilhaft durch das gesamte Inhalatorgehäuse 11 sichergestellt.

Der Inhalator 27 umfasst des Weiteren einen elektrischen Energiespeicher 14 und eine elektronische Steuerungsvorrichtung 15. Der Energiespeicher 14 ist in der Regel in dem Basisteil 16 angeordnet und kann insbesondere eine elektrochemische Einweg-Batterie oder ein wiederaufladbarer elektrochemischer Akku, beispielsweise ein Lithium-Ionen-Akku, sein. Die elektronische Steuerungsvorrichtung 15 umfasst mindestens eine digitale Datenverarbeitungseinrichtung, insbesondere Mikroprozessor und/oder Microcontroller, in dem Basisteil 16 (wie in Figur 3 gezeigt) und/oder in der Verbrauchseinheit 17.

In dem Inhalatorgehäuse 11 ist vorteilhaft ein Sensor, beispielsweise ein Drucksensor oder ein Druck- oder Strömungsschalter, angeordnet, wobei die Steuerungsvorrichtung 15 auf der Grundlage eines von dem Sensor ausgegebenen Sensorsignals feststellen kann, dass ein Konsument am Mundende 32 des Zigarettenprodukts 10 zieht, um zu inhalieren. In diesem Fall steuert die Steuerungsvorrichtung 15 den Schichtaufbau 20 an, um Flüssigkeit 50 aus dem Flüssigkeitsspeicher 18 als Aerosol/Dampf in den Luftstrom 34 zuzugeben.

Die in dem Flüssigkeitsspeicher 18 gespeicherte, zu dosierende Flüssigkeit 50 ist beispielsweise eine Mischung aus 1,2-Propylenglykol, Glycerin, Wasser, mindestens einem Aroma (Flavour) und/oder mindestens einem Wirkstoff, insbesondere Nikotin und/oder therapeutischen Wirkstoffen.

Die Verbrauchseinheit 100 umfasst vorteilhaft einen nichtflüchtigen Datenspeicher zum Speichern von die Verbrauchseinheit 100 betreffender Information bzw. Parameter. Der Datenspeicher kann Teil der elektronischen Steuerungsvorrichtung 15 sein. In dem Datenspeicher ist vorteilhaft Information zur Zusammensetzung der in dem Flüssigkeitsspeicher 18 gespeicherten Flüssigkeit 50, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verbrauchseinheit 100, Seriennummer, Herstelldatum und/oder Ablaufdatum, und/oder Zugzahl (Anzahl der Inhalationszüge durch den Konsumenten) bzw. der Nutzungszeit gespeichert. Der Datenspeicher ist vorteilhaft über Kontakte und/oder Leitungen mit der Steuereinrichtung 15 verbunden oder verbindbar.

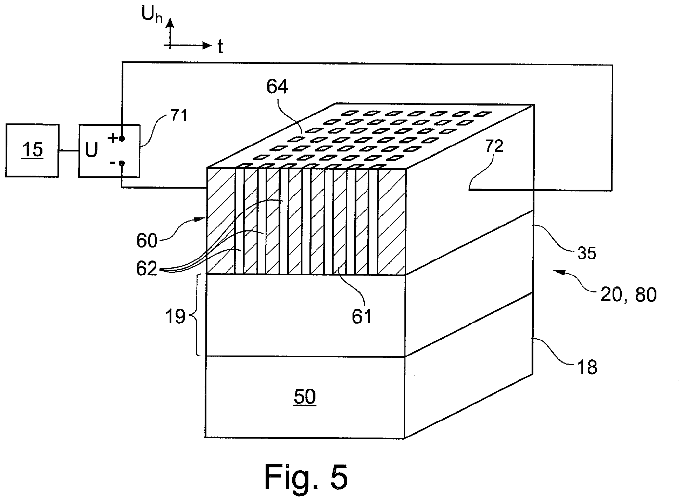

Figuren 4 und 5 zeigen eine vorteilhafte Ausführungsform einer Verdampfereinheit beziehungsweise eines als Schichtaufbau ausgebildeten Heizkörper-Dochtstruktur-Aufbaus 20. Die Verdampfereinheit beziehungsweise der Schichtaufbau 20 umfasst einen blockförmigen, vorzugsweise monolithischen Heizkörper 60 vorzugsweise aus einem elektrisch leitenden Material, vorzugsweise dotiertem Silizium, dotierter Keramik, Metall-Keramik, Filter-Keramik, Halbleiter, insbesondere Germanium, Graphit, Halbmetall und/oder Metall. Es ist nicht erforderlich, dass der gesamte Heizkörper 60 aus einem elektrisch leitenden Material besteht. Es kann beispielsweise ausreichen, dass die Oberfläche des Heizkörpers 60 elektrisch leitend, beispielsweise metallisch, beschichtet ist. In diesem Fall muss nicht die gesamte Oberfläche beschichtet sein, beispielsweise können Leiterbahnen auf einem nichtleitenden Grundkörper vorgesehen sein. Es ist auch nicht zwingend erforderlich, dass der gesamte Heizkörper 60 heizt; es kann beispielsweise ausreichen, wenn ein Abschnitt oder eine Heizschicht des Heizkörpers 60 im Bereich der Austrittsseite 64 heizt.

Der Heizkörper 60 ist mit einer Mehrzahl von Mikrokanälen beziehungsweise Durchgangskanälen 62 versehen, die eine Einlassseite 61 des Heizkörpers 60 mit einer Auslassseite 64 flüssigkeitsleitend verbinden. Die Einlassseite 61 ist über eine Dochtstruktur 19 flüssigkeitsleitend mit dem Flüssigkeitsspeicher 18 verbunden. Die Dochtstruktur 19 dient zur passiven Förderung von Flüssigkeit aus einem Flüssigkeitsspeicher 50 zu dem Heizkörper 60 mittels Kapillarkräften.

Der mittlere Durchmesser der Durchgangskanäle 62 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 61 in einen Durchgangskanal 62 eindringende Flüssigkeit durch den Durchgangskanal 62 nach oben steigt, bis der Durchgangskanal 62 mit Flüssigkeit gefüllt ist. Das Volumenverhältnis von Durchgangskanälen 62 zu Heizkörper 60, das als Porösität des Heizkörpers 60 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen und/oder der Durchmesser in einer nicht gezeigten Ausführungsform der mit Durchgangskanälen 62 versehenen Flächen des Heizkörpers 60 liegen beispielsweise im Bereich zwischen 0,5 mm und 3 mm, vorzugsweise zwischen 0,5 mm und 1 mm. Die Abmessungen der mit Durchgangskanälen 62 versehenen Flächen des Heizkörpers 60 können beispielsweise betragen: 0,95 mm x 1,75 mm oder 1,9 mm x 1,75 mm oder 1,9 mm x 0,75 mm. Die Kantenlängen und/oder der Durchmesser in einer nicht gezeigten Ausführungsform des Heizkörpers 60 können beispielsweise im Bereich zwischen 0,5 mm und 5 mm liegen, vorzugsweise im Bereich zwischen 0,75 mm und 4 mm, weiter vorzugsweise im Bereich zwischen 1 mm und 3 mm liegen. Die Fläche des Heizkörpers 60 (chip size) kann beispielsweise 1 mm x 3 mm, 2 mm x 2 mm oder 2 mm x 3 mm betragen.

Die Breite beziehungsweise der Durchmesser des Heizkörpers 60 liegt vorzugsweise im Bereich zwischen 1 mm und 5 mm, weiter vorzugsweise im Bereich zwischen 2 mm und 4 mm, und beträgt beispielsweise 3 mm. Die Höhe des Heizkörpers 60 liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm.

Die Anzahl der Durchgangskanäle 62 liegt vorzugsweise im Bereich zwischen vier und 1000. Auf diese Weise lässt sich der Wärmeeintrag von dem Substrat in die Durchgangskanäle 62 optimieren und eine gesicherte hohe Verdampfungsleistung sowie eine ausreichend große Dampfaustrittsfläche realisieren.

Die Durchgangskanäle 62 sind in Form eines quadratischen, rechteckigen, vieleckigen, runden, ovalen oder anders geformten Arrays angeordnet, wie in Figur 5 ersichtlich ist. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Durchgangskanäle 62 mit gesichert hoher Verdampfungsleistung realisieren.

Der Querschnitt der Durchgangskanäle 62 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Durchgangskanals 62 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 400 µm und beträgt beispielsweise 300 µm. Auf diese Weise lässt sich eine optimale Flüssigkeitsaufnahme und Portionsbildung bei ausreichend gutem Wärmeeintrag von dem Heizkörper 60 in die Durchgangskanäle 62 realisieren.

Der Abstand zweier Durchgangskanäle 62 beträgt vorzugsweise mindestens das 1,3-fache des lichten Durchmessers eines Durchgangskanals 62, wobei der Abstand auf die Mittelachsen der beiden Durchgangskanäle 62 bezogen ist. Der Abstand kann bevorzugt das 1,5- bis 5-fache, weiter bevorzugt das 2- bis 4-fache des lichten Durchmessers eines Durchgangskanals 62 betragen. Auf diese Weise lässt sich ein optimaler Wärmeeintrag von dem Substrat in die Durchgangskanäle und eine ausreichend stabile Anordnung und Wandstärke der Durchgangskanäle realisieren.

Aufgrund der vorbeschriebenen Merkmale kann der Heizkörper 60 auch als Volumenheizer bezeichnet werden.

Der Schichtaufbau 20 weist eine vorzugsweise von der Steuerungsvorrichtung 15 steuerbare Heizspannungsquelle 71 auf, die über Elektroden 72 an gegenüberliegenden Seiten des Heizkörpers 60 mit diesem verbunden ist, so dass eine von der Heizspannungsquelle 71 erzeugte elektrische Spannung Uh zu einem Stromfluss durch den Heizkörper 60 führt. Aufgrund des Ohm'schen Widerstands des elektrisch leitenden Heizkörpers 60 führt der Stromfluss zu einer Erhitzung des Heizkörpers 60 und daher zu einer Verdampfung von in den Durchgangskanälen 62 enthaltener Flüssigkeit. Der Heizkörper 60 wirkt somit als Verdampfer. Auf diese Weise erzeugter Dampf/Aerosol entweicht zur Auslassseite 64 aus den Durchgangskanälen 62 und wird der Luftströmung 34 beigemischt, siehe Figur 3. Genauer steuert bei Feststellung eines durch Ziehen des Konsumenten verursachten Luftstroms 34 durch den Luftkanal 30 die Steuerungsvorrichtung 15 die Heizspannungsquelle 71 an, wobei durch spontane Erhitzung die in den Durchgangskanälen 62 befindliche Flüssigkeit in Form von Dampf/Aerosol aus den Durchgangskanälen 62 getrieben wird.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Vorzugsweise ist in dem Datenspeicher des Inhalators 10 eine dem verwendeten Flüssigkeitsgemisch angepasste Spannungskurve Uh(t) hinterlegt, siehe Figur 5. Dies ermöglicht es, den Spannungsverlauf Uh(t) dem verwendeten Liquid angepasst vorzugeben, so dass sich die Heiztemperatur des Heizkörpers 60, und damit auch die Temperatur der kapillaren Durchgangskanäle 62, gemäß der bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt beispielsweise im Bereich zwischen 100° C und 400° C, bevorzugt zwischen 150° C und 350° C, weiter bevorzugt zwischen 190° C und 290° C. In manchen Anwendungen, beispielsweise medizinischen, kann eine Verdampfungstemperatur von weniger als 100° C vorgesehen sein, um beispielsweise Proteine und/oder Wirkstoffe ohne Schädigung verabreichen zu können.

An der Einlassseite 61 des Heizkörpers 60 ist eine poröse und/oder kapillare, flüssigkeitsleitende Dochtstruktur 19 angeordnet. Die Dochtstruktur 19 kontaktiert die Einlassseite 61 des Heizkörpers 60 flächig und deckt sämtliche Durchgangskanäle 62 einlassseitig ab, wie in den Figuren 4 und 5 ersichtlich ist. An der dem Heizkörper 60 gegenüberliegenden Seite ist die Dochtstruktur 19 flüssigkeitsleitend mit dem Flüssigkeitsspeicher verbunden. Die direkte Anbindung des Flüssigkeitsspeichers 18 an die Dochtstruktur 19 ist nur beispielhaft zu verstehen. Insbesondere können eine Flüssigkeitsschnittstelle und/oder eine mehrere Flüssigkeitsleitungen zwischen Flüssigkeitsspeicher 18 und Dochtstruktur 19 vorgesehen sein. Der Flüssigkeitsspeicher 18 kann daher auch beabstandet von der Dochtstruktur 19 angeordnet sein. Der Flüssigkeitsspeicher 18 kann in seinen Abmessungen größer als die Dochtstruktur 19 sein, siehe beispielsweise Figur 6. Die Dochtstruktur 19 kann beispielsweise in eine Öffnung eines Tankgehäuses 102 des Flüssigkeitsspeichers 18 eingesetzt sein. Es kann auch eine Mehrzahl von Schichtaufbauten 20 einem Flüssigkeitsspeicher 18 zugeordnet sein.

Die Dochtstruktur 19 besteht aus porösem und/oder kapillarem Material, das aufgrund von Kapillarkräften in der Lage ist, von dem Heizkörper 60 verdampfte Flüssigkeit in ausreichender Menge von dem Flüssigkeitsspeicher 18 zu dem Heizkörper 60 passiv nachzufördern, um ein Leerlaufen der Durchgangskanäle 62 und sich daraus ergebende Probleme zu verhindern.

Die Dochtstruktur 19 besteht vorteilhaft aus einem nichtleitenden Material, um eine unerwünschte Erwärmung von Flüssigkeit in die Dochtstruktur 19 durch Stromfluss zu vermeiden. Die Dochtstruktur 19 besteht vorteilhaft aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehr der vorgenannter Materialien. In einer vorteilhaften praktischen Ausführungsform kann die Dochtstruktur 19 mindestens ein Keramikfaserpapier und/oder eine poröse Keramik umfassen. Das Volumen der Dochtstruktur 19 liegt vorzugsweise im Bereich zwischen 1 mm³ und 10 mm³, weiter vorzugsweise im Bereich zwischen 2 mm³ und 8 mm³, noch weiter vorzugsweise im Bereich zwischen 3 mm³ und 7 mm³ und beträgt beispielsweise 5 mm³.

Falls die Dochtstruktur 19 aus einem leitenden Material besteht, was nicht ausgeschlossen ist, ist zwischen der Dochtstruktur 19 und dem Heizkörper 60 vorteilhaft eine Isolierschicht aus einem elektrisch und/oder thermisch isolierenden Material, beispielsweise Glas, Keramik oder Kunststoff, mit sich durch die Isolierschicht erstreckenden, mit den Durchgangskanälen 62 korrespondierenden Durchgangsöffnungen vorgesehen.

Die Größe der Poren bzw. Kapillaren in dem Material der Dochtstruktur 19 unterliegt vorteilhaft bestimmten Vorgaben. So ist die mittlere Poren-/Kapillargröße Dw von Poren oder Kapillaren der Dochtstruktur 19 im Kontaktbereich 35, 61 zu dem Heizkörper 60 vorteilhaft minimal, d.h. Dw = Pmin, und/oder vorteilhaft kleiner, vorzugsweise um mindestens den Faktor 2, weiter vorzugsweise um mindestens den Faktor 5, als der kleinste Abstand Dp zweier Durchgangskanäle 62 zueinander, d.h. Dw << Dp. Des Weiteren ist die mittlere Poren-/Kapillargröße Dw von Poren oder Kapillaren der Dochtstruktur 19 im Kontaktbereich 35, 61 zu dem Heizkörper 60 vorteilhaft kleiner, vorzugsweise um mindestens den Faktor 2, weiter vorzugsweise um mindestens den Faktor 5, als der kleinste lichte Durchmesser Dpw eines Durchgangskanals 62, d.h. Dw << Dpw.

Die Dochtstruktur 19 im Kontaktbereich 35, 61 zu dem Heizkörper 60 dient dazu, Flüssigkeit gleichmäßig zu verteilen, temperaturbeständig zu sein und mit ihren relativ kleinen Poren und/oder dünnen Kapillaren eine Art Rückschlagventil zu bilden, um unerwünschtes Rückfließen von blasenhaltiger Flüssigkeit aus dem Heizkörper 60 in die Dochtstruktur 19 und/oder in den Flüssigkeitsspeicher 18 zu verhindern.

In der Ausführungsform gemäß Figur 4 weist die Dochtstruktur 19 zwei beispielsweise planare Schichten 35, 36 auf, nämlich eine an der Einlassseite 61 des Heizkörpers 60 flächig anliegende und diese kontaktierende Dochtschicht 35, die als Kontaktschicht bezeichnet werden kann, und eine daran angrenzende Dochtschicht 36, die flüssigkeitsleitend mit dem Flüssigkeitsspeicher 18 verbunden ist und als entferntere Dochtschicht bezeichnet werden kann.

Die Kontaktschicht 35 weist eine in sich im Wesentlichen konstante Poren-/Kapillargrößenverteilung und eine in sich im Wesentlichen konstante mittlere Poren-/Kapillargröße Dw auf, die signifikant kleiner als der kleinste Abstand Dp zweier Durchgangskanäle 62 zueinander und signifikant kleiner als der kleinste lichte Durchmesser Dpw eines Durchgangskanals 62 ist: Dw << Dp, Dpw.

Die entferntere Dochtschicht 36 weist eine in sich im Wesentlichen konstante Poren-/Kapillargrößenverteilung und eine in sich im Wesentlichen konstante mittlere Poren-/Kapillargröße Dw' auf, die signifikant größer ist als die mittlere Poren-/Kapillargröße Dw der Kontaktschicht 35: Dw' > Dw, aber bevorzugt immer noch kleiner als Dp und/oder Dpw: Dw' < Dp, Dpw.

In einer vorteilhaften praktischen Ausführungsform kann die Kontaktschicht 35 beispielsweise eine Faserpapier- oder Keramikpapierschicht und/oder die Schicht 36 eine poröse Keramik sein.

Selbstverständlich kann die Dochtstruktur 19 mehr als zwei Dochtschichten 35, 36, ... aufweisen. Auch im Falle von mehr als zwei Dochtschichten 35, 36, ... wird die mittlere Poren-/Kapillargröße mit abnehmendem Abstand zu dem Heizkörper 60 vorteilhaft monoton (d.h. von Dochtschicht zu Dochtschicht) geringer und/oder bleibt gleich, und nimmt daher jedenfalls nicht zu.

In der Ausführungsform gemäß Figur 5 besteht die Dochtstruktur 19 nur aus einer Schicht, deren mittlere Poren-/Kapillargröße mit abnehmendem Abstand zu dem Heizkörper 60 monoton geringer wird.

In sämtlichen Ausführungsformen kann der gewünschte Poren- /Kapillargrößengradient optimal eingestellt und die Flüssigkeitsströmung zum Heizkörper 60 hin verlangsamt und vergleichmäßigt werden.

Die geschilderte Verringerung der mittleren Poren-/Kapillargröße in der Dochtstruktur 19 mit abnehmendem Abstand zum Heizkörper 60 gilt in der Richtung senkrecht zur Einlassseite 61 des Heizkörpers, d.h. senkrecht zur Kontaktfläche zwischen Heizkörper 60 und Dochtstruktur 19, bzw. parallel zum Verlauf der Durchgangskanäle 62. Innerhalb einer Sicht mit gleichem Abstand d zu dem Heizkörper 60 ist die mittlere Poren-/Kapillargröße in der Dochtstruktur 19 dagegen vorteilhaft konstant, damit sämtliche Durchgangskanäle 62 des Heizkörpers 60 gleichmäßig mit Flüssigkeit versorgt werden.

Die Durchgangskanäle 62 sind vorzugsweise mit ihrer Längsachse quer zu den Schichten 19, 35, 36 bzw. allgemeiner zu einer beliebigen Schichtenfolge angeordnet. Auf diese Weise lässt sich ein optimaler Wärmeeintrag von dem Heizkörper 60 in die Durchgangskanäle 62 realisieren.

Der Heizkörper 60 kann vorteilhaft aus Teilstücken eines Wafers mit Dünnfilmschichttechnologie hergestellt werden, welcher eine Schichtdicke von vorzugsweise kleiner oder gleich 1000 µm, weiter vorzugsweise 750 µm, noch weiter vorzugsweise kleiner oder gleich 500 µm und vorteilhaft 300 µm aufweist. Oberflächen des Heizkörpers 60 können vorteilhaft hydrophil sein. Die Einlassseite 61 und/oder die Auslassseite 64 des Heizkörpers 60 kann vorteilhaft mikrostrukturiert sein bzw. Mikroausnehmungen (micro grooves) aufweisen.

Der Heizkörper-Dochtstruktur-Aufbau 20 ist so eingestellt, dass eine Flüssigkeitsmenge vorzugsweise im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann der Heizkörper-Dochtstruktur-Aufbau 20 hinsichtlich der Flüssigkeits-/Dampfmenge pro Zug, d.h. je Zugdauer von 0,5 s bis 5 s, bevorzugt von 1 s bis 3 s, einstellbar sein.

In dem Ausführungsbeispiel gemäß Figur 6 umfasst die Dochtstruktur 19 mehr als zwei, hier vier Schichten. Unmittelbar angrenzend an den Heizkörper 60, und diesen flächig kontaktierend, ist eine Filterschicht 55 angeordnet, die insbesondere aus einer, zwei oder mehr Mikroglasfaser-Lagen bestehen kann. Daran flächig angrenzend kann eine Faserpapier-Schicht 56 angeordnet sein. Daran flächig angrenzend sind vorteilhaft Dochtschichten 57, 58 vorgesehen, beispielsweise eine Keramikdocht-Schicht 57 und eine Öllampendocht-Schicht 58, d.h. ein Glasfaser-Dochtmaterial, das herkömmlich für die Dochte von Öllampen verwendet wird.

In der Ausführungsform nach Figur 6 erfüllt mindestens die an dem Heizkörper 60 flächig anliegende Schicht 55 vorteilhaft die zuvor erläuterten Bedingungen für die Poren-/Kapillargröße Dw << Dp, Dpw. Vorteilhaft kann auch die Schicht 57 und/oder die Schicht 58 diese Bedingungen erfüllen. Des Weiteren können die Kapillarkräfte für die Kapillarförderung der Flüssigkeit von dem Flüssigkeitsspeicher 18 zu dem Heizkörper 60 überwiegend oder vollständig von den Dochtschichten 57, 58 bereitgestellt werden. Es ist im Allgemeinen nicht erforderlich, wenn sämtliche Schichten der Dochtstruktur 19 Kapillarkräfte für die Kapillarförderung der Flüssigkeit bereitstellen. Es kann auch ausreichen, dass nur eine Schicht der Dochtstruktur 19 Kapillarkräfte für die Kapillarförderung der Flüssigkeit bereitstellt.

Der Heizkörper-Dochtstruktur-Aufbau 20 weist vorteilhaft einen insbesondere plattenförmigen Träger 23 zum Halten des Heizkörpers 60 und/oder der Dochtstruktur 19 auf, wie in den Figuren 6 bis 8 gezeigt ist. Der Träger 23 kann aus einem geeigneten Material, beispielsweise Keramik, Glas und/oder Kunststoff einschließlich faserverstärktem Kunststoff, beispielsweise Leiterplattenmaterial bestehen und weist eine Durchgangsöffnung 25 auf, durch die sich die Dochtstruktur 19 erstreckt und in der die Dochtstruktur 19 gehalten ist.

Die Dicke des Trägers 23 liegt vorzugsweise im Bereich zwischen 0,5 mm bis 4 mm, weiter vorzugsweise im Bereich zwischen 1 mm bis 3 mm, noch weiter vorzugsweise im Bereich zwischen 1 mm und 2 mm und kann beispielsweise 1,6 mm oder 2 mm betragen. Die Dicke einer in der Durchgangsöffnung 25 des Trägers 23 angeordneten Dochtschicht 57 kann an die Dicke des Trägers 23 angepasst sein bzw. dieser entsprechen und daher beispielsweise ebenfalls 1,6 mm oder 2 mm betragen.

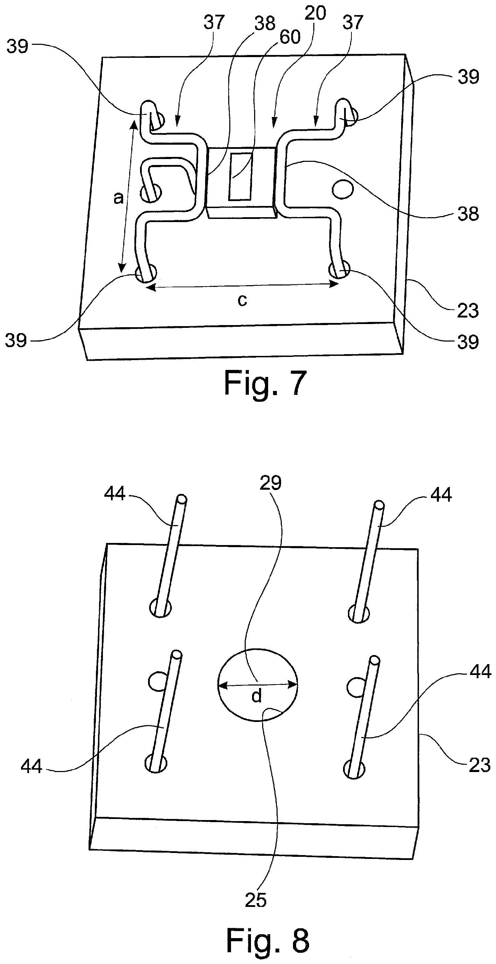

Die Durchgangsöffnung 25 ist vorteilhaft kreisrund, was einfach zu fertigen ist. Der Durchmesser d, oder ggf. der mittlere Durchmesser, der Durchgangsöffnung 25 (siehe Figur 8) liegt vorzugsweise im Bereich zwischen 0,5 mm und 4 mm, vorzugsweise im Bereich zwischen 1 mm bis 3 mm, weiter vorzugsweise im Bereich zwischen 1,5 mm und 2,5 mm und beträgt beispielsweise 2 mm.

Der Durchmesser d der Durchgangsöffnung 25 ist kleiner oder gleich, vorteilhaft kleiner als die Breite beziehungsweise des Durchmessers b des Heizkörpers 60, siehe Figur 8. Das Volumen der Durchgangsöffnung 25, bzw. das Dochtvolumen in der Durchgangsöffnung 25, liegt vorteilhaft im Bereich zwischen 1 mm³ und 8 mm³, vorzugsweise im Bereich zwischen 2 mm³ und 6,5 mm³, weiter vorzugsweise im Bereich zwischen 2,5 mm³ und 5 mm³.

Die Dochtstruktur 19 kann im freien, vormontierten Zustand ein Übermaß, d.h. einen größeren Durchmesser haben als die Durchgangsöffnung 25, um zusätzliche Haltekräfte des Schafts 29 in der Durchgangsöffnung 25 zu erzeugen.

Der Durchmesser der Dochtschicht 35 ist vorteilhaft größer als der Durchmesser d der Durchgangsöffnung 25. Vorzugsweise steht die Dochtschicht 35 über ihren gesamten Umfang über die Durchgangsöffnung 25 mit Überstand über. Aufgrund des allseitigen Überstands die Dochtschicht 35 über die Durchgangsöffnung 25 wird bei einer Klemmung des Heizkörpers 60 auf den Träger 23 die Dochtschicht 35, und somit die gesamte Dochtstruktur 19, sicher in dem Heizkörper-Dochtstruktur-Aufbau 20 gehalten.

Die Klemmung des Heizkörpers 60 auf dem Träger 23 wird mittels mindestens zwei Klemmelementen 37 bewirkt, siehe insbesondere Figur 7, die an gegenüberliegenden Seiten des Heizkörpers 60 an diesem angreifen. Jedes Klemmelement 37 weist vorteilhaft einen Klemmbügel 38 auf, der an zwei voneinander beabstandeten Befestigungspunkten 39 federnd an dem Träger 23 befestigt ist und eine Vorspannung erzeugt, mittels der der Heizkörper 60 und der Teller 28 auf dem Träger 23 festgeklemmt und somit sicher gehalten wird.

Der Abstand a der beiden Befestigungspunkte 39 eines Klemmbügels 38 voneinander liegt vorzugsweise im Bereich zwischen 4 mm und 10 mm, weiter vorzugsweise im Bereich zwischen 5 mm bis 8 mm und beträgt beispielsweise 6 mm. Der Abstand c zwischen den Befestigungspunkten 39 zweier Klemmbügel 39 voneinander liegt vorzugsweise im Bereich zwischen 5 mm und 12 mm, weiter vorzugsweise im Bereich zwischen 6 mm bis 10 mm und beträgt beispielsweise 8 mm. Die Abmessungen des beispielsweise rechteckigen Trägers 23 liegen vorzugsweise im Bereich zwischen 6 mm und 20 mm, weiter vorzugsweise im Bereich zwischen 8 mm bis 17 mm und noch weiter vorzugsweise im Bereich zwischen 10 mm und 14 mm.

Besonders vorteilhaft dienen die Klemmelemente 37 gleichzeitig als Elektroden zur Kontaktierung des Heizkörpers 60 und dessen Versorgung mit Heizstrom. Zu diesem Zweck bestehen die Klemmelemente 37 bzw. die Klemmbügel 38 vorteilhaft aus einem elektrisch leitenden Material, beispielsweise kann es sich um Metalldraht, beispielsweise Messingdraht handeln. Aufgrund der Linienkontaktierung zwischen dem Klemmbügel 38 und dem Heizkörper 60 ergibt sich eine ausgezeichnete elektrische Verbindung zwischen dem Klemmelement 37 und dem Heizkörper 60, bei gleichzeitig idealer thermischer Entkopplung zwischen dem Klemmelement 37 und dem Heizkörper 60 wegen fehlendem Flächenkontakt. Wärmedissipation von dem Heizkörper 60 in das Klemmelement 37 ist daher gering, die Elektroden 38 bleiben signifikant kühler als der Heizkörper 60. Dioden-Effekte werden vermieden und rein Ohm'scher Ladungsträgertransport ermöglicht.

Der Klemmbügel 38 kann den Heizkörper 60 seitlich parallel zur Auslassseite 64 und/oder senkrecht auf die Auslassseite 64 und/oder in einer Ecknut mit einem Zwischenwinkel, beispielsweise zwischen 30° und 60°, sowohl seitlich als auch senkrecht auf die Auslassseite 64 klemmen. Die letztgenannte Möglichkeit involviert zwei Kontaktlinien zwischen dem Klemmbügel und dem Heizkörper 60, was die elektrische Kontaktierung weiter verbessert. Ein Klemmelement 37 kann auch mehr als einen Klemmbügel 38 aufweisen, insbesondere beliebige zwei oder alle drei der Klemmbügel.

Die Klemmelemente 37 sind mittels elektrischer Leitungen 12 vorteilhaft mit einer in der Verbrauchseinheit 17 vorgesehenen Leiterplatte 26 (PCB) verbunden, um die elektrische Verbindung zu der elektronischen Steuerungsvorrichtung 15 und zu der Energiequelle 46 für die Stromversorgung des Heizkörpers 60 herzustellen. Auf der Leiterplatte 26 sind vorteilhaft elektronische Komponenten der Verbrauchseinheit 17 angeordnet.

Die Leiterplatte 26 ist in dem Ausführungsbeispiel gemäß Figur 6 ein separates Teil und von dem Träger 23 beabstandet auf dessen dem Heizkörper 60 abgewandter Seite 43 angeordnet. Die Leiterplatte 26 weist eine Durchgangsöffnung 10 auf, durch die sich die Dochtstruktur 19 erstreckt und in der die Dochtstruktur 19 gehalten ist. Die elektrischen Leitungen 12 umfassen hier beispielsweise vier Metallstifte 44, die auf der Seite 33 des Trägers 23 in den Befestigungspunkten 39 mit den Klemmelementen 37 verbunden und jeweils durch eine Durchgangsbohrung 45 durch den Träger 23 hindurchgeführt sind und dann auf der abgewandten Seite 43 den Abstand zwischen dem Träger 23 und der Leiterplatte 26 überbrücken.

In einer anderen Ausführungsform kann der Träger 23 die Leiterplatte 26 ausbilden. Die elektrischen Leitungen 12 können dann entfallen. Es ist auch möglich, dass der Heizkörper-Dochtstruktur-Aufbau 20 selbst keine Leiterplatte umfasst, sondern die Klemmbügel 38 beispielsweise über flexible isolierte Leitungen 12, oder auf andere geeignete Weise, mit einer etwa in dem Basisteil 16 angeordneten Leiterplatte verbunden sind.

An der Unterseite 43 des Trägers 23 kann ein Dichtelement 73, beispielsweise ein Dichtring, zur Abdichtung des Trägers 23 gegen ein Gehäuse des Flüssigkeitsspeichers 18, oder einer anderen unter dem Träger 23 angeordneten Komponente, angeordnet sein.

In Figur 6 ist eine Kanüle 82 gezeigt, durch welche sich die Dochtstruktur 19, insbesondere die Dochtschicht 58 beziehungsweise der Schaft 29 erstreckt. Die Kanüle 82 ist in diesem Beispiel von der Leiterplatte 26 gehalten, beispielsweise indem die Kanüle 82 mit einem an der Befestigungsseite 84 angeordneten Abschnitt des Mantels 90 in die Durchgangsöffnung 10 geklemmt ist.

Die Dochtschicht 58 liegt umfänglich an der Innenseite 91 des Mantels 90 an und erstreckt sich durch den Kanal 93 der Kanüle 82.

Im Folgenden wird der Ablauf des Verdampfungsvorgangs erläutert.

In einem Ausgangszustand ist die Spannungsquelle 71 für den Heizvorgang ausgeschaltet.

Zum Verdampfen von Flüssigkeit 50 wird die Spannungsquelle 71 für den Heizkörper 60 aktiviert. Die Spannung Uh wird dabei so eingestellt, dass die Verdampfungstemperatur in dem Heizkörper 60 und somit in den Durchgangskanälen 62 an das individuelle Verdampfungsverhalten des eingesetzten Flüssigkeitsgemischs angepasst ist. Dies verhindert die Gefahr von lokaler Überhitzung und dadurch Schadstoffentstehung.

Sobald eine Flüssigkeitsmenge verdampft ist, die dem Volumen der Durchgangskanäle 62 entspricht oder damit in Zusammenhang steht, wird die Heizspannungsquelle 71 deaktiviert. Da die Liquideigenschaften und -menge vorteilhaft exakt bekannt sind und der Heizkörper 60 einen messbaren temperaturabhängigen Widerstand aufweist, kann dieser Zeitpunkt sehr genau bestimmt bzw. gesteuert werden. Die Energieaufnahme des Heizkörper-Dochtstruktur-Aufbaus 20 lässt sich daher gegenüber bekannten Vorrichtungen reduzieren, da die benötigte Verdampfungsenergie dosierter und damit exakter eingebracht werden kann.

Nach Abschluss des Heizvorgangs sind die Durchgangskanäle 62 überwiegend oder vollständig entleert. Die Heizspannung 71 wird dann so lange ausgeschaltet gehalten, bis mittels Nachförderung von Flüssigkeit durch die Dochtstruktur 19 die Durchgangskanäle 62 wieder aufgefüllt sind. Sobald dies der Fall ist, kann der nächste Heizzyklus durch einschalten der Heizspannung 71 begonnen werden.

Die von der Heizspannungsquelle 71 erzeugte Ansteuerfrequenz des Heizkörpers 60 liegt im Allgemeinen vorteilhaft im Bereich von 1 Hz bis 50 kHz, bevorzugt im Bereich von 30 Hz bis 30 kHz, noch weiter vorteilhaft im Bereich von 100 Hz bis 25 kHz.

Die Frequenz und der Tastgrad der Heizspannung Uh für den Heizkörper 60 sind vorteilhaft an die Eigenschwingung bzw. Eigenfrequenz der Blasenschwingungen während der Blasensiedung angepasst. Vorteilhaft kann die Periodendauer 1/f der Heizspannung daher im Bereich zwischen 1 ms und 50 ms, weiter vorteilhaft zwischen 10 ms und 40 ms, noch weiter vorteilhaft zwischen 15 ms und 30 ms liegen und beispielsweise 20 ms betragen. Je nach Zusammensetzung der verdampften Flüssigkeit können andere als die genannten Frequenzen optimal an die Eigenschwingung bzw. Eigenfrequenz der Blasenschwingungen angepasst sein.

Des Weiteren hat sich gezeigt, dass der durch die Heizspannung Uh erzeugten maximale Heizstrom vorzugsweise nicht mehr als 7 A, weiter vorzugsweise nicht mehr als 6,5 A, noch weiter vorzugsweise nicht mehr als 6 A betragen und optimalerweise im Bereich zwischen 2 A und 6 A liegen sollten, um konzentrierten Dampf bei Vermeidung von Überhitzung zu gewährleisten. Der Heizstrom kann eine Untergrenze von beispielsweise 1 A, vorzugsweise 2 A, haben, um eine ausreichende Verdampfung gewährleisten zu können.

Die Förderrate der Dochtstruktur 19 ist wiederum optimal an die an die Verdampfungsrate des Heizkörpers 60 angepasst, so dass jederzeit ausreichend Flüssigkeit nachgefördert werden kann und ein Leerlaufen des Bereichs vor dem Heizkörper 60 vermieden wird.

Der Heizkörper-Dochtstruktur-Aufbau 20 ist vorzugsweise auf der Grundlage von MEMS-Technologie, insbesondere aus dotiertem Silizium, gefertigt und daher vorteilhaft ein Mikro-Elektro-Mechanisches System.

Vorgeschlagen wird nach dem zuvor Gesagten vorteilhaft ein Heizkörper-Dochtstruktur-Aufbau 20 bestehend aus einem vorteilhaft mindestens auf der Einlassseite 61 planaren Heizkörper 60 auf Si-Basis und einer oder mehrerer darunter liegender Kapillarstrukturen 19 mit vorteilhaft unterschiedlicher Porengröße. Die direkt an der Einlassseite 61 des Heizkörpers 60 angeordnete Dochtstruktur 19 verhindert die Bildung von Blasen an der Einlassseite 61 des Heizkörpers 60, da Gasblasen eine weitere Förderwirkung unterbinden und gleichzeitig zu einer (lokalen) Überhitzung des Heizkörpers 60 aufgrund fehlender Kühlung durch nachströmendes Liquid führen.

## Patentansprüche

1. Verdampferkopf (1) für einen Inhalator (27), insbesondere für ein elektronisches Zigarettenprodukt, umfassend
- einen Heizkörper-Dochtstruktur-Aufbau (20) aufweisend einen elektrisch betreibbaren Heizkörper (60) mit mindestens einem flüssigkeitsleitenden Durchgangskanal (62) und einer an einer Einlassseite (61) des Heizkörpers (60) angeordneten porösen und/oder kapillaren Dochtstruktur (19), und
- eine Trägerplatte (23) zum Halten des Heizkörper-Dochtstruktur-Aufbaus (20), **dadurch gekennzeichnet, dass**
- der Verdampferkopf (1) eine flüssigkeitsleitende Kanüle (82) aufweist, die einen Einstechbereich (83) vorzugsweise vorragend aufweist, wobei
- der Einstechbereich (83) zum Einstechen in ein externes Teil, insbesondere in einen Flüssigkeitsspeicher (18), ausgebildet ist.

2. Verdampferkopf (1) nach Anspruch 1, **dadurch gekennzeichnet, dass**
- die Dochtstruktur (19) und/oder der Heizkörper (60) an einer Befestigungsseite (84) der Kanüle (82) angeordnet ist.

3. Verdampferkopf (1) nach einem der vorangehenden Ansprüche, **dadurch gekennzeichnet, dass**
- die Dochtstruktur (19) wenigstens teilweise innerhalb der Kanüle (82) angeordnet ist.

4. Verdampferkopf (1) nach einem der vorangehenden Ansprüche, **dadurch gekennzeichnet, dass**
- der Heizkörper-Dochtstruktur-Aufbau (20) als ein Schichtaufbau ausgebildet ist.

5. Verdampferkopf (1) nach einem der vorangehenden Ansprüche, **dadurch gekennzeichnet, dass**
- die Kanüle (82) einen sich zwischen einer Befestigungsseite (84) und einer Stichseite (85) erstreckenden Mantel (90) aufweist, wobei
- der Mantel (90) wenigstens teilweise den Heizkörper-Dochtstruktur-Aufbau (20) umfänglich, insbesondere abschließend, ummantelt.

6. Verdampferkopf (1) nach einem der vorangehenden Ansprüche, **dadurch gekennzeichnet, dass**
- der Einstechbereich (83) eine Schneide (86) aufweist.

7. Verdampferkopf (1) nach einem der vorangehenden Ansprüche, **dadurch gekennzeichnet, dass**
- die Trägerplatte (23) eine Durchgangsöffnung (25) aufweist.

8. Verdampferkopf (1) nach Anspruch 7, **dadurch gekennzeichnet, dass**
- sich der Heizkörper-Dochtstruktur-Aufbau (20) mindestens teilweise durch die Durchgangsöffnung (25) der Trägerplatte (23) erstreckt.

9. Verdampferkopf (1) nach einem der vorangehenden Ansprüche, **dadurch gekennzeichnet, dass**
- der Verdampferkopf (1) ein Arretierungselement (87) zur Arretierung in einem externen Teil, insbesondere in einem Flüssigkeitsspeicher (18), umfasst.

10. Verdampferkopf (1) nach einem der vorangehenden Ansprüche, **dadurch gekennzeichnet, dass**
- ein Mundstück (101) an dem Verdampferkopf (1), insbesondere an der Trägerplatte (23), befestigbar ist.

11. Verdampferkopf (1) nach einem der vorangehenden Ansprüche, **dadurch gekennzeichnet, dass**
- die Kanüle (82) aus Metall besteht.

12. Verdampferkopf (1) nach einem der vorangehenden Ansprüche, **dadurch gekennzeichnet, dass**
- der Heizkörper (60) ein Mikromechanisches System (MEMS) und/oder siliziumbasiert ist.

13. Verbrauchseinheit (100), umfassend einen Verdampferkopf (1) nach einem der Ansprüche 1 bis 12 und einen Flüssigkeitsspeicher (18) mit einem Tankgehäuse (102), wobei das Tankgehäuse (102) von einer Kanüle (82) durchstechbar ausgebildet ist, **dadurch gekennzeichnet, dass**
- der Verdampferkopf (1) mit der Kanüle (82) in den Flüssigkeitsspeicher (18) zur Herstellung einer gebrauchsfertigen Verbrauchseinheit (100) einstechbar ist.

14. Verbrauchseinheit (100) nach Anspruch 13, **dadurch gekennzeichnet, dass** die Verbrauchseinheit (100) ein Mundstück (101) aufweist.

15. Verbrauchseinheit (100) nach Anspruch 14, **dadurch gekennzeichnet, dass**
- die Trägerplatte (23) des Verdampferkopfes (1) an dem Mundstück (101) befestigt ist.

16. Verbrauchseinheit (100) nach einem der Ansprüche 13 bis 15, **dadurch gekennzeichnet, dass**
- die Kanüle (82) im gebrauchsfertigen Zustand in dem Mundstück (101) versenkt ist.

17. Verbrauchseinheit (100) nach einem der Ansprüche 13 bis 16, **dadurch gekennzeichnet, dass**
- die Trägerplatte (23) im gebrauchsfertigen Zustand mit dem Tankgehäuse (102) des Flüssigkeitsspeichers (18) fluchtet.

18. Verbrauchseinheit (100) nach einem der Ansprüche 13 bis 17, **dadurch gekennzeichnet, dass**
- der Verdampferkopf (1) im gebrauchsfertigen Zustand den Flüssigkeitstank (104) flüssigkeitsdicht abschließt.

19. Verbrauchseinheit (100) nach einem der Ansprüche 13 bis 18, **dadurch gekennzeichnet, dass**
- die Trägerplatte (23) das Tankgehäuse (102) des Flüssigkeitsspeichers (18) im gebrauchsfertigen Zustand zu einem vollständigen und flüssigkeitsdichten Flüssigkeitstank (104) ergänzt.

20. Verbrauchseinheit (100) nach einem der Ansprüche 13 bis 19, **dadurch gekennzeichnet, dass** das Tankgehäuse (102) des Flüssigkeitsspeichers (18) ein Einstechsiegel (103) umfasst.

21. Verbrauchseinheit (100) nach einem der Ansprüche 13 bis 20, **dadurch gekennzeichnet, dass** der Flüssigkeitsspeicher (18) wenigstens teilweise aus von einer Kanüle (82) durchstechbarem Kunststoff und/oder Metall, insbesondere Aluminium, gebildet ist.

22. Verbrauchseinheit (100) nach einem der Ansprüche 13 bis 21, **dadurch gekennzeichnet, dass** der Flüssigkeitsspeicher (18) eine Arretierungseinrichtung (105) zur Arretierung, insbesondere durch eine Drehbewegung, eines externen Teils, insbesondere eines Verdampferkopfes (1), umfasst.

23. Inhalator (27) mit einem Verdampferkopf (1) und/oder einer Verbrauchseinheit (100) nach einem der vorangehenden Ansprüche, und einem Basisteil (16) mit einem elektrischen Energiespeicher (14) zur Versorgung elektrischer Komponenten des Inhalators (27).

24. Verfahren zur Herstellung einer gebrauchsfertigen Verbrauchseinheit (100) für einen Inhalator (27), **gekennzeichnet durch** die folgenden Schritte:
- Bereitstellung eines Verdampferkopfes (1) nach einem der Ansprüche 1 bis 12;
- Bereitstellung eines Flüssigkeitsspeichers (18) mit einem Tankgehäuse (102), wobei das Tankgehäuse (102) von einer Kanüle (82) durchstechbar ausgebildet ist;
- Einstechen der Kanüle (82) des Verdampferkopfes (1) in das Tankgehäuse (102) des Flüssigkeitsspeichers (18).

25. Verfahren zur Herstellung einer gebrauchsfertigen Verbrauchseinheit (100) nach Anspruch 24, **gekennzeichnet durch** den folgenden Schritt:
- Arretierung des Verdampferkopfes (1) am Flüssigkeitsspeicher (18), insbesondere **durch** eine Drehbewegung.

## Claims

1. Vaporiser head (1) for an inhaler (27), in particular for an electronic cigarette product, comprising
- a heating element-wick structure assembly (20) having an electrically operable heating element (60), which has at least one liquid-conducting through-channel (62) and a porous and/or capillary wick structure (19) arranged at an inlet side (61) of the heating element (60), and
- a support plate (23) for retaining the heating element-wick structure assembly (20), **characterised in that**
- the vaporiser head (1) has a liquid-conducting cannula (82) having a piercing region (83) preferably protruding, wherein
- the piercing region (83) is designed for piercing an external component, in particular a liquid reservoir (18).

2. Vaporiser head (1) according to claim 1, **characterised in that**
- the wick structure (19) and/or the heating element (60) is/are arranged on an attachment side (84) of the cannula (82).

3. Vaporiser head (1) according to any of the preceding claims, **characterised in that**
- the wick structure (19) is arranged at least in part inside the cannula (82).

4. Vaporiser head (1) according to any of the preceding claims, **characterised in that**
- the heating element-wick structure assembly (20) is formed as a layered assembly.

5. Vaporiser head (1) according to any of the preceding claims, **characterised in that**
- the cannula (82) has a sheath (90) extending between an attachment side (84) and a pricking side (85),
- the sheath (90) circumferentially encasing, in particular closing, the heating element-wick structure assembly (20) at least in part.

6. Vaporiser head (1) according to any of the preceding claims, **characterised in that**
- the piercing region (83) has a cutting edge (86).

7. Vaporiser head (1) according to any of the preceding claims, **characterised in that**
- the support plate (23) has a through-opening (25).

8. Vaporiser head (1) according to claim 7, **characterised in that**
- the heating element-wick structure assembly (20) extends at least in part through the through-opening (25) in the support plate (23).

9. Vaporiser head (1) according to any of the preceding claims, **characterised in that**
- the vaporiser head (1) comprises a locking member (87) for locking in an external component, in particular in a liquid reservoir (18).

10. Vaporiser head (1) according to any of the preceding claims, **characterised in that**
- a mouthpiece (101) can be attached to the vaporiser head (1), in particular to the support plate (23).

11. Vaporiser head (1) according to any of the preceding claims, **characterised in that**
- the cannula (82) consists of metal.

12. Vaporiser head (1) according to any of the preceding claims, **characterised in that**
- the heating element (60) is a microelectromechanical system (MEMS) and/or is silicon-based.

13. Consumption unit (100) comprising a vaporiser head (1) according to any of claims 1 to 12 and a liquid reservoir (18) with a tank housing (102), wherein the tank housing (102) can be punctured by a cannula (82),
**characterised in that**
- the vaporiser head (1) can pierce the liquid reservoir (18) by means of the cannula (82) to produce a ready-to-use consumption unit (100).

14. Consumption unit (100) according to claim 13, **characterised in that** the consumption unit (100) has a mouthpiece (101).

15. Consumption unit (100) according to claim 14, **characterised in that**
- the support plate (23) of the vaporiser head (1) is attached to the mouthpiece (101).

16. Consumption unit (100) according to any of claims 13 to 15, **characterised in that**
- in the ready-to-use state, the cannula (82) is sunk into the mouthpiece (101).

17. Consumption unit (100) according to any of claims 13 to 16, **characterised in that**
- in the ready-to-use state, the support plate (23) is aligned with the tank housing (102) of the liquid reservoir (18).

18. Consumption unit (100) according to any of claims 13 to 17, **characterised in that**
- in the ready-to-use state, the vaporiser head (1) closes the liquid tank (104) in a liquid-tight manner.

19. Consumption unit (100) according to any of claims 13 to 18, **characterised in that**
- in the ready-to-use state, the support plate (23) supplements the tank housing (102) of the liquid reservoir (18) to form a complete and liquid-tight liquid tank (104).

20. Consumption unit (100) according to any of claims 13 to 19, **characterised in that**
- the tank housing (102) of the liquid reservoir (18) comprises a piercing seal (103).

21. Consumption unit (100) according to any of claims 13 to 20, **characterised in that**
- the liquid reservoir (18) is made at least in part of plastics material and/or metal, in particular aluminium, capable of being punctured by a cannula (82).

22. Consumption unit (100) according to any of claims 13 to 21, **characterised in that**
- the liquid reservoir (18) comprises a locking device (105) for locking an external component, in particular a vaporiser head (1), in place, in particular by means of a rotational movement.

23. Inhaler (27) comprising a vaporiser head (1) and/or a consumption unit (100) according to any of the preceding claims, and a basic part (16) having an electrical energy storage device (14) for powering electrical components of the inhaler (27).

24. Method for producing a ready-to-use consumption unit (100) for an inhaler (27), **characterised by** the following steps:
- providing a vaporiser head (1) according to any of claims 1 to 12;
- providing a liquid reservoir (18) with a tank housing (102), wherein the tank housing (102) can be punctured by a cannula (82);
- piercing the tank housing (102) of the liquid reservoir (18) using the cannula (82) of the vaporiser head (1).

25. Method for producing a ready-to-use consumption unit (100) according to claim 24, **characterised by** the following step:
- locking the vaporiser head (1) in place on the liquid reservoir (18), in particular by means of a rotational movement.

## Revendications

1. Tête d'évaporateur (1) pour un inhalateur (27), en particulier pour un produit sous forme de cigarette électronique, comprenant :
- une construction corps chauffant-structure de mèche (20) présentant un corps chauffant (60) pouvant être actionné électriquement avec au moins un canal de passage (62) conducteur de liquide et une structure de mèche (19) poreuse et/ou capillaire disposée sur un côté entrée (61) du corps chauffant (60), et
- une plaque de support (23) pour maintenir la construction corps chauffant-structure de mèche (20),
**caractérisée en ce**
- **que** la tête d'évaporateur (1) présente une canule (82) conductrice de liquide, qui présente une zone de piquage (83) de préférence en saillie, dans laquelle
- la zone de piquage (83) est conçue pour le piquage dans une partie externe, en particulier dans un accumulateur de liquide (18).

2. Tête d'évaporateur (1) selon la revendication 1, **caractérisée en ce**
- **que** la structure de mèche (19) et/ou le corps chauffant (60) sont disposés sur un côté fixation (84) de la canule (82).

3. Tête d'évaporateur (1) selon l'une des revendications précédentes, **caractérisée en ce**
- **que** la structure de mèche (19) est disposée au moins partiellement à l'intérieur de la canule (82).

4. Tête d'évaporateur (1) selon l'une des revendications précédentes, **caractérisée en ce**
- **que** la construction corps chauffant-structure de mèche (20) est réalisée sous la forme d'une construction en couches.

5. Tête d'évaporateur (1) selon l'une des revendications précédentes, **caractérisée en ce**
- **que** la canule (82) présente une enveloppe (90) s'étendant entre un côté fixation (84) et un côté piquage (85), dans laquelle
- l'enveloppe (90) enveloppe au moins partiellement la construction corps chauffant-structure de mèche (20) sur sa périphérie, en particulier de manière à la fermer.

6. Tête d'évaporateur (1) selon l'une des revendications précédentes, **caractérisée en ce**
- **que** la zone de piquage (83) présente un tranchant (86).

7. Tête d'évaporateur (1) selon l'une des revendications précédentes, **caractérisée en ce**
- **que** la plaque de support (23) présente une ouverture de passage (25).

8. Tête d'évaporateur (1) selon la revendication 7, **caractérisée en ce**
- **que** la construction corps chauffant-structure de mèche (20) s'étend au moins partiellement à travers l'ouverture de passage (25) de la plaque de support (23).

9. Tête d'évaporateur (1) selon l'une des revendications précédentes, **caractérisée en ce**
- **que** la tête d'évaporateur (1) comprend un élément de blocage (87) pour le blocage dans une partie externe, en particulier dans un accumulateur de liquide (18).

10. Tête d'évaporateur (1) selon l'une des revendications précédentes, **caractérisée en ce**
- **qu'**un embout buccal (101) peut être fixé à la tête d'évaporateur (1), en particulier à la plaque de support (23).

11. Tête d'évaporateur (1) selon l'une des revendications précédentes, **caractérisée en ce**
- **que** la canule (82) est en métal.

12. Tête d'évaporateur (1) selon l'une des revendications précédentes, **caractérisée en ce que**
- le corps chauffant (60) est un système micromécanique (MEMS) et/ou à base de silicium.

13. Unité consommable (100), comprenant une tête d'évaporateur (1) selon l'une des revendications 1 à 12 et un accumulateur de liquide (18) avec un boîtier de réservoir (102), le boîtier de réservoir (102) étant conçu de manière à pouvoir être transpercé par une canule (82), **caractérisée en ce**
- **que** la tête d'évaporateur (1) peut être piquée avec la canule (82) dans l'accumulateur de liquide (18) pour fabriquer une unité consommable (100) prête à l'emploi.

14. Unité consommable (100) selon la revendication 13, **caractérisée en ce que** l'unité consommable (100) présente un embout buccal (101).

15. Unité consommable (100) selon la revendication 14, **caractérisée en ce**
- **que** la plaque de support (23) de la tête d'évaporateur (1) est fixée à l'embout buccal (101).

16. Unité consommable (100) selon l'une des revendications 13 à 15, **caractérisée en ce**
- **que** la canule (82) est escamotée dans l'embout buccal (101) à l'état prêt à l'emploi.

17. Unité consommable (100) selon l'une des revendications 13 à 16, **caractérisée en ce**
- **que** la plaque de support (23) est alignée avec le boîtier de réservoir (102) de l'accumulateur de liquide (18) à l'état prêt à l'emploi.

18. Unité consommable (100) selon l'une des revendications 13 à 17, **caractérisée en ce**
- **que** la tête d'évaporateur (1) obture le réservoir de liquide (104) de manière étanche aux liquides à l'état prêt à l'emploi.

19. Unité consommable (100) selon l'une des revendications 13 à 18, **caractérisée en ce**
- **que** la plaque de support (23) complète le boîtier de réservoir (102) de l'accumulateur de liquide (18) à l'état prêt à l'emploi pour former un réservoir de liquide (104) complet et étanche aux liquides.

20. Unité consommable (100) selon l'une des revendications 13 à 19, **caractérisée en ce que** le boîtier de réservoir (102) de l'accumulateur de liquide (18) comprend un sceau de piquage (103).

21. Unité consommable (100) selon l'une des revendications 13 à 20, **caractérisée en ce que** l'accumulateur de liquide (18) est formé au moins en partie de matière plastique et/ou de métal, en particulier d'aluminium, pouvant être transpercé par une canule (82).

22. Unité consommable (100) selon l'une des revendications 13 à 21, **caractérisée en ce que** l'accumulateur de liquide (18) comprend un moyen de blocage (105) pour bloquer, en particulier par un mouvement de rotation, une partie externe, en particulier une tête d'évaporateur (1).

23. Inhalateur (27) comprenant une tête d'évaporateur (1) et/ou une unité consommable (100) selon l'une des revendications précédentes, et une partie de base (16) avec un accumulateur d'énergie électrique (14) pour l'alimentation des composants électriques de l'inhalateur (27).

24. Procédé de fabrication d'une unité consommable (100) prête à l'emploi pour un inhalateur (27), **caractérisé par** les étapes suivantes :
- mise à disposition d'une tête d'évaporateur (1) selon l'une des revendications 1 à 12;
- mise à disposition d'un accumulateur de liquide (18) avec un boîtier de réservoir (102), le boîtier de réservoir (102) étant conçu de manière à pouvoir être transpercé par une canule (82) ;
- piquage de la canule (82) de la tête d'évaporateur (1) dans le boîtier de réservoir (102) de l'accumulateur de liquide (18).

25. Procédé de fabrication d'une unité consommable (100) prête à l'emploi selon la revendication 24, **caractérisé par** l'étape suivante :
- blocage de la tête d'évaporateur (1) sur l'accumulateur de liquide (18), en particulier par un mouvement de rotation.
